# EUROPEAN PATENT APPLICATION

(11) **EP 3 266 422 A1**
(43) Date of publication of application: **10.01.2018**
(21) Application number: 16759053.8
(22) Date of filing: 04.03.2016
(51) Int. Cl.: A61F 2/68, B25J 11/00

(54) **MUSCULAR STRENGTH ASSISTANCE DEVICE**

(30) Priority: 05.03.2015 JP 2015043932
(71) Applicant: Nabtesco Corporation, Tokyo 102-0093 (JP); Chuo University, Tokyo 192-0393 (JP)
(72) Inventor: KIKUTANI, Isao, Tokyo 102-0093 (JP); IMAMURA, Hiroya, Tokyo 102-0093 (JP); YOKOYAMA, Kazuya, Tokyo 102-0093 (JP); NAKAMURA, Taro, Tokyo 112-8551 (JP); INOSE, Hiroki, Tokyo 1128551 (JP)
(74) Representative: Grünecker Patent- und Rechtsanwälte PartG mbB
(86) International application number: PCT/JP2016/056887
(87) International publication number: WO 2016/140361

(57) **Abstract**

One object is to ensure a moment of a desired magnitude for assisting in an activity of moving two body parts relative to each other, while keeping an output of an actuator low, the actuator generating a force at a point of action of the moment. A muscular strength assisting apparatus 1 includes a first assistance unit 10 having a first actuator 11 for changing a distance between a first body-side disposition portion 11A mounted to a first part of a body and a first outer-side disposition portion 11B separable from and contactable with the first body-side disposition portion 11A, and a second assistance unit 20 having a second actuator 21 for changing a distance between a second body-side disposition portion 21A mounted to a second part of the body and a second outer-side disposition portion 21B separable from and contactable with the second body-side disposition portion 21A. The muscular strength assisting apparatus 1 is mounted to the body so that a direction connecting the second body-side disposition portion 21A to the second outer-side disposition portion 21B is non-parallel to a direction connecting the first body-side disposition portion 11A to the first outer-side disposition portion 11B.

## Description

### TECHNICAL FIELD

The present invention relates to a muscular strength assisting apparatus.

### BACKGROUND

A muscular strength assisting apparatus is attached to a body of a human in order to assist the human in his/her activities. For example, Japanese Patent Application Publication No. 2008-67762A discloses a muscular strength assisting apparatus including an elastic member having one end portion attached to a shoulder part of a human and the other end portion attached to a lower body of the human and thus extending along a back part of the human, and an electric wire winding machine (hereinafter, abbreviated as a winding machine) that expands the elastic member in a longitudinal direction.

In the muscular strength assisting apparatus of Japanese Patent Application Publication No. 2008-67762A, when the human has taken a forward bending posture, the elastic member is wound by the winding machine and thus is expanded Thus, an elastic force is generated in the elastic member, and a muscle bad for maintaining the forward bending posture applied to a lower back part of the human can be reduced by the elastic force. Furthermore, according to this type of muscular strength assisting apparatus, the elastic force generated at this time acts to assist the human in an activity of returning to an upright posture, and thus a muscle load applied to the lower back part of the human when recovering to the upright posture can also be reduced Moreover, when the human has returned from the forward bending posture to the upright posture, the elastic member is unwound by the winding machine, and thus the elastic force of the elastic member is lost. This can also prevent a muscle load from being undesirably generated due to the elastic force of the elastic member when the human is in the upright posture.

### SUMMARY

In the above-mentioned muscular strength assisting apparatus of Japanese Patent Application Publication No. 2008-67762A, the winding machine is provided on a lower back part of a human, and the one end portion of the elastic member is attached to a shoulder part of the human, while the other end portion of the elastic member is connected to the winding machine on a rear side of the lower back part. When the human has taken a forward bending posture, the winding machine functions to wind the elastic member so that the elastic member is expanded, and an elastic force of the elastic member generated by the expansion assists the human in an activity of returning to an upright posture. This elastic force generates a moment about a joint point between the lower back part and a back part of the human as a rotation axis, thus assisting the human in the activity of returning to the upright posture. This reduces a muscle load applied to the lower back part of the human when recovering to the upright posture.

In the muscular strength assisting apparatus of Japanese Patent Application Publication No. 2008-67762A, however, the other end portion of the elastic member and the winding machine are connected to each other at a position relatively close to a lower back part of a human, so that a moment arm of a moment about a joint point between the lower back part and a back part of the human as a rotation axis is relatively smalL Because of this, in order to ensure a moment of a desired magnitude for sufficiently assisting the human in an activity of returning from a forward bending posture to an upright posture, it may be required to expand the elastic member to a large extent so that an elastic force of the elastic member is increased It may, therefore, be required to increase an output of the winding machine. This might lead to a size increase of the winding machine, resulting in a possibility of a size increase of the apparatus as a whole and a weight increase of the apparatus as a whole.

The present invention has been devised in view of the above-described actual circumstances and has as its object to provide a muscular strength assisting apparatus capable of ensuring a moment of a desired magnitude for assisting in an activity of moving two different body parts relative to each other, while keeping an output of an actuator low, the actuator generating a force at a point of action of said moment, and thus capable of suppressing a size increase and a weight increase of the apparatus as a whole.

The present invention relates to a muscular strength assisting apparatus provided with a first assistance unit having a first actuator including a first body-side disposition portion mounted to a first part of a body and a first outer-side disposition portion separable from and contactable with the first body-side disposition portion, the first actuator being capable of changing a distance between the first body-side disposition portion and the first outer-side disposition portion, and a second assistance unit having a second actuator including a second body-side disposition portion mounted to a second part of the body and a second outer-side disposition portion separable from and contactable with the second body-side disposition portion, the second actuator being capable of changing a distance between the second body-side disposition portion and the second outer-side disposition portion, the second outer-side disposition portion being mountable to the first outer-side disposition portion. The first assistance unit is mounted to the first part and the second assistance unit is mounted to the second part so that a direction connecting the second body-side disposition portion to the second outer-side disposition portion of the second actuator is non-parallel to a direction connecting the first body-side disposition portion to the first outer-side disposition portion. The above-described feature of being "mounted to a first part of a body" refers to a state of being directly mounted to the first part of the body or a state of being indirectly mounted to the first part of the body. Specifically, being "directly mounted to the first part of the body" means, for example, that the first body-side disposition portion of the first actuator is mounted to a skeleton of a human. Furthermore, being "indirectly mounted to the first part of the body" means, for example, that the first body-side disposition portion of the first actuator is mounted to a part of a human body via an attachment tool attached to the part of the human body or mounted to a part of a human body via an attachment tool attached to the human body and a wire or the like connected to the attachment tooL Similarly to the above, the above-described feature of being "mounted to a second part of the body" also refers to a state of being directly mounted to the second part of the body or indirectly mounted to the second part of the body.

According to the present invention, the first assistance unit is mounted to the first part and the second assistance unit is mounted to the second part so that a direction connecting the second body-side disposition portion to the second outer-side disposition portion of the second actuator of the second assistance unit is non-parallel to a direction connecting the first body-side disposition portion to the first outer-side disposition portion of the first actuator of the first assistance unit. In this state, the first actuator changes a distance between the first body-side disposition portion and the first outer-side disposition portion. At this time, a force is generated in the first actuator, and with this force acting as a point of action, a moment for assisting in an activity of moving the first part and the second part relative to each other can be generated The moment thus generated can assist a human in his/her activities. Further, at this time, the second actuator increases a distance between the second body-side disposition portion and the second outer-side disposition portion of the second actuator and thus can increase a moment arm of the moment. Thus, in a state after the moment arm has thus been increased, even in a case where an output of the first actuator of the first assistance unit is kept lower than would be required for obtaining a moment of a desired magnitude in a state before the moment arm is increased, a moment equal in magnitude to the above-mentioned moment of a desired magnitude can be ensured Thus, according to the present invention, it is possible to ensure a moment of a desired magnitude for assisting in an activity of moving two different body parts relative to each other, while keeping an output of an actuator low, the actuator generating a force at a point of action of said moment, and thus to suppress a size increase and a weight increase of the apparatus as a whole.

Furthermore, in the muscular strength assisting apparatus of the present invention, preferably, in a state where the second actuator is stopped from operating, the second assistance unit is capable of maintaining a distance between the second body-side disposition portion and the second outer-side disposition portion at a predetermined length.

Furthermore, in the muscular strength assisting apparatus of the present invention, preferably, in a state where the first actuator is stopped from operating, the first assistance unit is capable of maintaining a distance between the first body-side disposition portion and the first outer-side disposition portion at a predetermined length.

Furthermore, in the muscular strength assisting apparatus of the present invention, it may also be possible that the first part represents a shoulder part or a thigh part, and the second part represents a lower back part.

Furthermore, preferably, the muscular strength assisting apparatus of the present invention is provided further with a control device for controlling the second actuator to operate so that, before the first actuator starts to operate, the second outer-side disposition portion of the second actuator moves away from the second part.

Furthermore, it may also be possible to adopt the following configuration. That is, the muscular strength assisting apparatus of the present invention is provided further with a third assistance unit having a third actuator including a third body-side disposition portion mounted to a third part of the body and a third outer-side disposition portion separable from and contactable with the third body-side disposition portion, the third actuator being capable of changing a distance between the third body-side disposition portion and the third outer-side disposition portion, the third outer-side disposition portion being mountable to the second outer-side disposition portion. The third assistance unit is mounted to the third part so that a direction connecting the second body-side disposition portion to the second outer-side disposition portion of the second actuator is non-parallel to a direction connecting the third body-side disposition portion to the third outer-side disposition portion of the third actuator. Similarly to the above-mentioned cases of the first part and the second part, the above-described feature of being "mounted to a third part of the body" also refers to a state of being directly mounted to the third part of the body or indirectly mounted to the third part of the body.

In this case, preferably, in a state where the third actuator is stopped from operating, the third assistance unit is capable of maintaining a distance between the third body-side disposition portion and the third outer-side disposition portion at a predetermined length.

Furthermore, in this case, it may also be possible that the first part represents a shoulder part, the second part represents a lower back part, and the third part represents a thigh part.

Furthermore, in this case, preferably, the muscular strength assisting apparatus of the present invention is provided further with a control device for controlling the second actuator to operate so that, before the first actuator and the third actuator start to operate, the second outer-side disposition portion of the second actuator moves away from the second part.

### ADVANTAGES

According to the present invention, it is possible to ensure a moment of a desired magnitude for assisting in an activity of moving two different body parts relative to each other, while keeping an output of an actuator low, the actuator generating a force at a point of action of said moment, and thus to suppress a size increase and a weight increase of the apparatus as a whole.

### BRIEF DESCRIPTION OF THE DRAWINGS

Fig. 1 is a side view showing a state where a muscular strength assisting apparatus according to one embodiment of the present invention is attached to a human body.
Fig. 2 is a rear view showing a state where the muscular strength assisting apparatus shown in Fig. 1 is attached to the human body.
Fig. 3 is a front view showing a state where the muscular strength assisting apparatus shown in Fig. 1 is attached to the human body.
Fig. 4 is a perspective view showing a state where the muscular strength assisting apparatus shown in Fig. 1 is attached to the human body.
Fig. 5 is a schematic view of a system configuration of the muscular strength assisting apparatus shown in Fig. 1.
Fig. 6 is a flow chart explaining one example of an operation of the muscular strength assisting apparatus shown in Fig. 1.
Fig. 7 is a timing chart explaining one example of the operation of the muscular strength assisting apparatus shown in Fig. 1.
Fig. 8 is a view showing how the muscular strength assisting apparatus operates in accordance with the flow chart in Fig. 6.
Fig. 9 is a view showing how the muscular strength assisting apparatus operates in accordance with the flow chart in Fig. 6.
Fig. 10 is a view showing how the muscular strength assisting apparatus operates in accordance with the flow chart in Fig. 6.
Fig. 11 is a view showing how the muscular strength assisting apparatus operates in accordance with the flow chart in Fig. 6.
Fig. 12 is a view explaining a modification example of the muscular strength assisting apparatus shown in Fig. 1.
Fig. 13 is a view explaining a modification example of the muscular strength assisting apparatus shown in Fig. 1.
Fig. 14 is a view explaining a modification example of the muscular strength assisting apparatus shown in Fig. 1.
Fig. 15 is a view explaining a modification example of the muscular strength assisting apparatus shown in Fig. 1.
Fig. 16 is a view explaining a modification example of the muscular strength assisting apparatus shown in Fig. 1.
Fig. 17 is a view explaining a modification example of the muscular strength assisting apparatus shown in Fig. 1.
Fig. 18 is a view explaining a modification example of the muscular strength assisting apparatus shown in Fig. 1.
Fig. 19 is a view explaining a modification example of the muscular strength assisting apparatus shown in Fig. 1.
Fig. 20 is a view explaining a modification example of the muscular strength assisting apparatus shown in Fig. 1.
Fig. 21 is a view explaining a modification example of the muscular strength assisting apparatus shown in Fig. 1.
Fig. 22 is a view explaining a modification example of the muscular strength assisting apparatus shown in Fig. 1.
Fig. 23 is a view explaining a modification example of the muscular strength assisting apparatus shown in Fig. 1.
Fig. 24 is a view explaining a modification example of the muscular strength assisting apparatus shown in Fig. 1.
Fig. 25 is a view explaining a modification example of the muscular strength assisting apparatus shown in Fig. 1.
Fig. 26 is a view explaining a modification example of the muscular strength assisting apparatus shown in Fig. 1.
Fig. 27 is a view explaining a modification example of the muscular strength assisting apparatus shown in Fig. 1.
Fig. 28 is a view explaining a modification example of the muscular strength assisting apparatus shown in Fig. 1.
Fig. 29 is a view explaining a modification example of the muscular strength assisting apparatus shown in Fig. 1.
Fig. 30 is a view explaining a modification example of the muscular strength assisting apparatus shown in Fig.1.
Fig. 31 is a view explaining a modification example of the muscular strength assisting apparatus shown in Fig. 1.
Fig. 32 is a view explaining a modification example of the muscular strength assisting apparatus shown in Fig. 1.
Fig. 33 is a view explaining a modification example of the muscular strength assisting apparatus shown in Fig. 1.
Fig. 34 is a view explaining a modification example of the muscular strength assisting apparatus shown in Fig. 1.
Fig. 35 is a view explaining a modification example of the muscular strength assisting apparatus shown in Fig. 1.
Fig. 36 is a view explaining a modification example of the muscular strength assisting apparatus shown in Fig. 1.
Fig. 37 is a view explaining a modification example of the muscular strength assisting apparatus shown in Fig. 1.
Fig. 38A is a view explaining a modification example of the muscular strength assisting apparatus shown in Fig. 1.
Fig. 38B is a view explaining a modification example of the muscular strength assisting apparatus shown in Fig. 1.

### DESCRIPTION OF THE PREFERRED EMBODIMENTS

With reference to the appended drawings, the following describes one embodiment of the present invention.

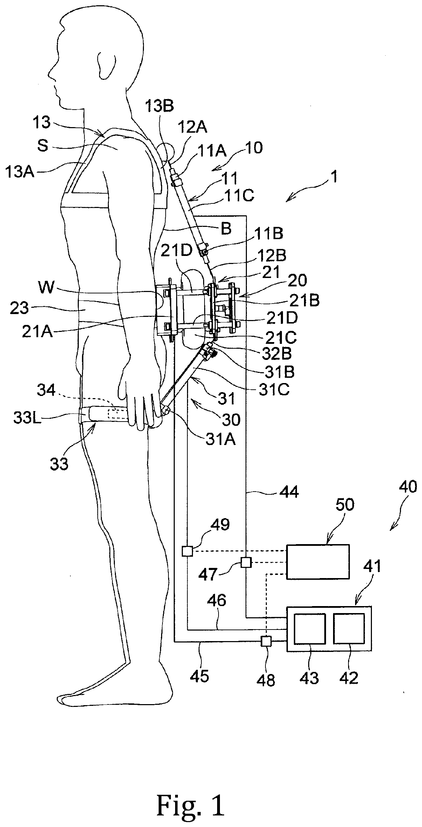

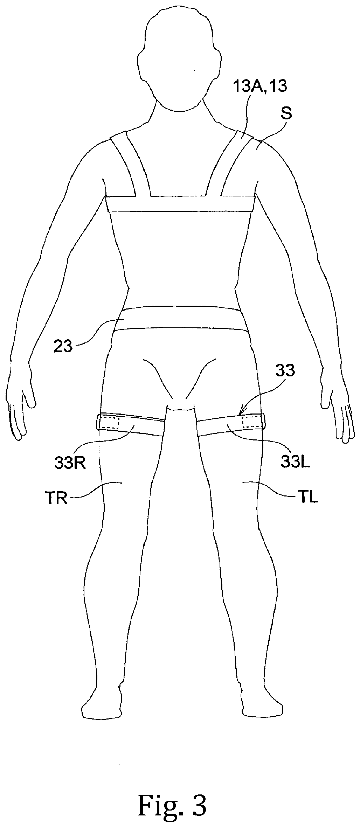

Fig. 1 to Fig. 4 show a state where a muscular strength assisting apparatus 1 according to one embodiment of the present invention is attached to a human body. The muscular strength assisting apparatus 1 may be provided with a first assistance unit 10, a second assistance unit 20, a third assistance unit 30, and a control unit 40 that controls operations of the assistance units 10, 20, and 30.

(First Assistance Unit] The first assistance unit 10 may have a first actuator 11 including a first body-side disposition portion 11A mounted to a part of a body (in this embodiment, a shoulder part S) and a first outer-side disposition portion 11B separable from and contactable with the first body-side disposition portion 11A and capable of changing a distance between the first body-side disposition portion 11A and the first outer-side disposition portion 11B. The first actuator 11 in this embodiment may have the first body-side disposition portion 11A, the first outer-side disposition portion 11B, and a tubular portion 11C formed of an elastic member joining the first body-side disposition portion 11A to the first outer-side disposition portion 11B.

The first actuator 11 may be a fluid pouring type actuator (a so-called artificial muscle type actuator] and switch between a state where a fluid flows into an inside of the tubular portion 11 and a state where the fluid is discharged therefrom so that the tubular portion 11C is elastically deformed, thus being capable of changing a distance between the first body-side disposition portion 11A and the first outer-side disposition portion 11B. As the above-mentioned fluid caused to flow into the inside of the tubular portion 11, air may be used in this embodiment. Said air may be supplied from an air supply portion 41 (see Fig. 1) in the control unit 40.

As shown in the figures, the first body-side disposition portion 11A may be connected to a shoulder attachment tool 13 attached to the shoulder part S of an upper body of a human via a wire 12A. Thus, in the first assistance unit 10, the first body-side disposition portion 11A of the first actuator 11 may be indirectly mounted to the shoulder part S of the human. The wire 12A may be a metallic wire or the like and made of a material having a high elastic modulus. The shoulder attachment tool 13 may be made of, for example, cloth and have an over-the-shoulder portion 13A wrapped over the shoulder part S of the human and a joint portion 13B joined to the over-the-shoulder portion 13A on a back part B of the human. Of these, the joint portion 13B may have the wire 12A connected thereto. In this embodiment, an end portion of the wire 12A may be connected to the joint portion 13B swingably in an up-down direction and a left-right direction. As a way to realize such a connection mode, for example, an openable hook (such as a carabiner) provided at the end portion of the wire 12A may be mounted to a ring-shaped member provided in the joint portion 13B. In this embodiment, an upper body may refer to a part of a human body upper than a lower back part W (such as the shoulder part S or the back part B), and a lower body refers to a part of the human body lower than the lower back part W (such as a thigh part TL and TR, a lower leg part, or a foot part).

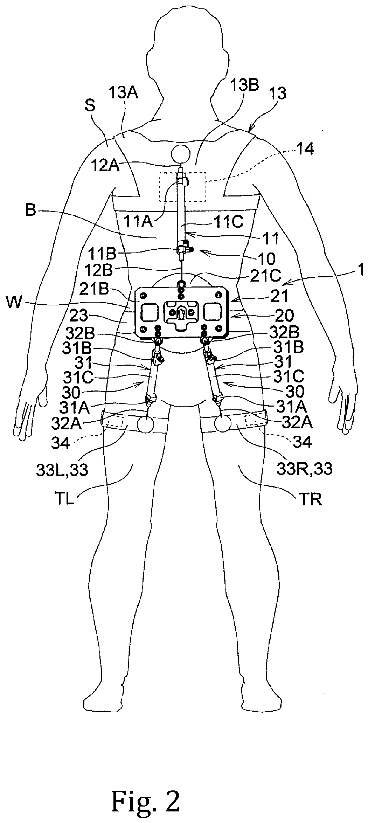

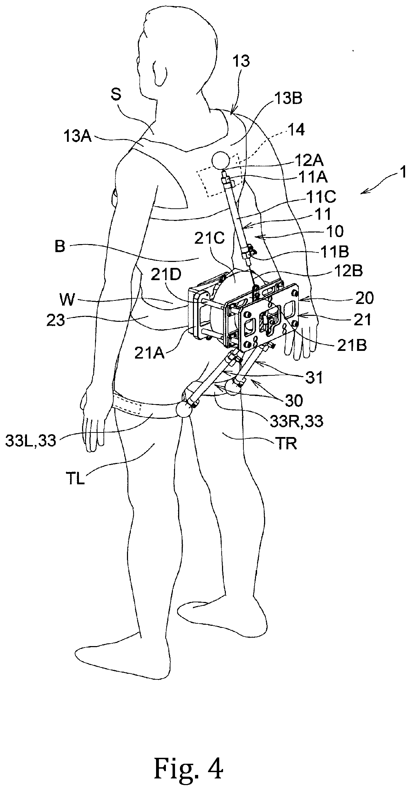

In this embodiment, a body posture detection sensor 14 (see Fig. 2 or Fig. 4) may be provided in the above-mentioned joint portion 13B, and the body posture detection sensor 14 may output, to the control unit 40, a signal indicating a degree of forward bending when a human shifts from an upright posture to a forward bending posture. As the body posture detection sensor 14, for example, an electromyography sensor, an angle sensor, an acceleration sensor, a strain gauge, or the like can be used In this embodiment, an "upright posture" may refer to such a posture that the back part B is not bent forward with respect to the lower back part W and the shoulder part S is positioned straight above the lower back part W.

On the other hand, the first outer-side disposition portion 11B may be mounted to the second assistance unit 20 via a wire 12B. Thus, in the first assistance unit 10, the first outer-side disposition portion 11B of the first actuator 11 may be indirectly mounted to the second assistance unit 20. Similarly to the wire 12A, the wire 12B may be a metallic wire or the like and made of a material having a high elastic modulus. An end portion of the wire 12B may also be connected to the second assistance unit 20 swingably in the up-down direction and the left-right direction. This connection mode will be detailed in a later description of the second assistance unit 20.

In the first assistance unit 10 described above, in a state before air from the air supply portion 41 in the control unit 40 is caused to flow into the above-mentioned tubular portion 11C in the first actuator 11, a distance between the first body-side disposition portion 11A and the first outer-side disposition portion 11B may be defined as a reference length. From this reference length state, air may be caused to flow into the inside of the tubular portion 11C, thus elastically deforming the tubular portion 11C so that the tubular portion 11C is radially inflated, and thus a length of the tubular portion 11C in an axial direction may be contracted. Thus, a distance between the first body-side disposition portion 11A and the first outer-side disposition portion 11B may become shorter than the reference length, so that a length of the first assistance unit 10 as a whole may become shorter than a length thereof as a whole before the air is caused to flow into the tubular portion 11C. A length of the first assistance unit 10 as a whole may refer to a length between the shoulder attachment tool 13 and the end portion of the wire 12B, which is connected to the second assistance unit 20.

On the other hand when the above-mentioned air caused to flow into the inside of the tubular portion 11C is discharged, the radially directed inflation of the tubular portion 11C may be released, so that a length of the tubular portion 11C in the axial direction may recover to a state before the inflation. Thus, a distance between the first body-side disposition portion 11A and the first outer-side disposition portion 11B may recover to the reference length, so that a length of the first assistance unit 10 as a whole may recover to a length thereof as a whole before the air is caused to flow into the tubular portion 11C.

Furthermore, in this embodiment, when air is caused to flow into the inside of the above-mentioned tubular portion 11C so that a length of the tubular portion 11C in the axial direction is contracted the first actuator 11 can be stopped from operating by, for example, restricting air inflow into and air discharge from the tubular portion 11C. Thus, in the first assistance unit 10, in a state where the first actuator 11 is stopped from operating, a distance between the first body-side disposition portion 11A and the first outer-side disposition portion 11B of said first actuator 11 can be maintained at a predetermined length. Thus, in a state where the first actuator 11 is stopped from operating, the first assistance unit 10 may be capable of maintaining a length thereof as a whole at a fixed value.

Furthermore, though not shown, a length adjustment mechanism for adjusting a length of the wire 12A may be provided between the wire 12A and the joint portion 13B of the shoulder attachment tool 13. Thus, a length of the wire 12A extending between the first body-side disposition portion 11A of the first actuator 11 and the shoulder attachment tool 13 may be adjustable, and thus the wire 12A can be adjusted to an appropriate length depending on a size of a human body. In a case where a length of the wire 12A is adjusted by the length adjustment mechanism, the wire 12A may be maintained at the length thus adjusted Thus, even when pulled by the first body-side disposition portion 11A of the first actuator 11, the wire 12A may be prevented from having a length different from an adjusted length. It may also be possible that the length adjustment mechanism described above is provided at the wire 12B.

(Second Assistance Unit) The second assistance unit 20 may have a second actuator 21 including a second body-side disposition portion 21A mounted to a part of a body (in this embodiment, the lower back part W) and a second outer-side disposition portion 21B separable from and contactable with the second body-side disposition portion 21A and capable of changing a distance between the second body-side disposition portion 21A and the second outer-side disposition portion 21B. The second actuator 21 in this embodiment may have the second body-side disposition portion 21A, the second outer-side disposition portion 21B, and a bellows portion 21C formed of an elastic member joining the second body-side disposition portion 21A to the second outer-side disposition portion 21B.

The second actuator 21 may switch between a state where a fluid flows into an inside of the bellows portion 21C (an accordion portion) having a fluid inflow space formed therein and a state where the fluid is discharged therefrom so that the bellows portion 21C is expanded/contracted, thus being capable of changing a distance between the second body-side disposition portion 21A and the second outer-side disposition portion 21B. As the above-mentioned fluid caused to flow into the inside of the bellows portion 21C, air may be used in this embodiment. Similarly to a case of the above-mentioned tubular portion 11C, said air may be supplied from the air supply portion 41 (see Fig. 1) in the control unit 40. Furthermore, in this embodiment, the second body-side disposition portion 21A and the second outer-side disposition portion 21B may be each formed in a plate shape, and a plurality of bar-shaped guide members 21D extending along an expansion/contraction direction of the bellows portion 21C may be provided in an outer peripheral portion of the second body-side disposition portion 21A. An outer peripheral portion of the second outer-side disposition portion 21B may be slidably inserted over the guide members 21D, and thus a relative movement between the second body-side disposition portion 21A and the second outer-side disposition portion 21B may be stabilized

As shown in the figures, the second body-side disposition portion 21A may be connected to a lower back attachment tool 23 attached to the lower back part W of a human. Thus, in the second assistance unit 20, the second body-side disposition portion 21A of the second actuator 21 may be indirectly mounted to the lower back part W of the human. The lower back attachment tool 23 may be made of, for example, cloth and wrapped from an abdomen part of the human over the lower back part W of the human. On the other hand, the second outer-side disposition portion 21B may be connected to the wire 12B of the first assistance unit 10. Thus, in the second assistance unit 20, the second outer-side disposition portion 21B of the second actuator 21 may be indirectly mounted to the first outer-side disposition portion 11B of the first actuator 11 of the first assistance unit 10. As mentioned above, in this embodiment, the end portion of the wire 12B of the first assistance unit 10 may be connected to the second assistance unit 20 swingably in the up-down direction and the left-right direction. Specifically, the end portion of the wire 12B may be connected to an upper portion of the second outer-side disposition portion 21B of the second assistance unit 20 swingably in the up-down direction and the left-right direction. As a way to realize such a connection mode, for example, an openable hook (such as a carabiner) provided at the end portion of the wire 12B may be mounted to a ring-shaped member provided in the second outer-side disposition portion 21B.

Furthermore, as shown in Fig. 1, in this embodiment, the second assistance unit 20 may be mounted to the lower back part W of a human via the lower back attachment tool 23 so that the second assistance unit 20 protrudes rearward from the lower back part W in a state where the expansion/contraction direction of the second actuator 21 is aligned with a front-back direction. In this state, on the back part B of the human, the first assistance unit 10 may extend from the second assistance unit 20 to the shoulder part S along the back part B of the human. That is, in this embodiment, the first assistance unit 10 may be mounted to the shoulder part S and the second assistance unit 20 may be mounted to the lower back part W so that a direction connecting the second body-side disposition portion 21A to the second outer-side disposition portion 21B of the second actuator 21 of the second assistance unit 20 is non-parallel to a direction connecting the first body-side disposition portion 11A to the first outer-side disposition portion 11B of the first actuator 11 of the first assistance unit 10. In this mounted state, preferably, in a case where a human having the muscular strength assisting apparatus 1 attached thereto is in a upright posture, the first assistance unit 10 may be adjusted to extend, while sagging, from the second assistance unit 20 to the shoulder part S of the human. In this case, the human can effortlessly take a forward bending posture.

Furthermore, in a state where, as mentioned above, the end portion of the wire 12B of the first assistance unit 10 is connected to the upper portion of the second outer-side disposition portion 21B of the second assistance unit 20, it can also be said that the first outer-side disposition portion 11B of the first actuator 11 of the first assistance unit 10 is indirectly mounted to the lower back part W of the human via the wire 12B and the second assistance unit 20 mounted to the lower back part W. Accordingly, the second assistance unit 20 may function as a mounting member for indirectly mounting the first outer-side disposition portion 11B of the first actuator 11 of the first assistance unit 10 to the lower back part W. At a position on a rear side of the lower back part W, the second assistance unit 20 functioning as the mounting member may connect the second outer-side disposition portion 21B thereof to the first outer-side disposition portion 11B of the first actuator 11 via the wire 12B, thus indirectly mounting the first outer-side disposition portion 11B of the first actuator 11 to the lower back part W.

In the second assistance unit 20 described above, in a state before air from the air supply portion 41 in the control unit 40 is caused to flow into the above-mentioned bellows portion 21C in the second actuator 21, a distance between the second body-side disposition portion 21A and the second outer-side disposition portion 21B may be defined as a reference length. From this reference length state, air may be caused to flow into the inside of the bellows portion 21C, and thus the bellows portion 21C may be expanded Thus, a length between the second body-side disposition portion 21A and the second outer-side disposition portion 21B may become longer than the reference length. On the other hand, when the above-mentioned air caused to flow into the inside of the bellows portion 21C is discharged, the bellows portion 21C may be contracted to a state before the expansion. Thus, a distance between the second body-side disposition portion 21A and the second outer-side disposition portion 21B may recover to the reference length.

Furthermore, in this embodiment, when air is caused to flow into the inside of the above-mentioned bellows portion 21C so that the bellows portion 21C is expanded, the second actuator 21 can be stopped from operating by, for example, restricting air inflow into and air discharge from the bellows portion 21C. Thus, in a state where the second actuator 21 is stopped from operating, the second assistance unit 20 may be capable of maintaining a distance between the second body-side disposition portion 21A and the second outer-side disposition portion 21B of said second actuator 21 at a predetermined length.

(Third Assistance Unit) The third assistance unit 30 may have a third actuator 31 including a third body-side disposition portion 31A mounted to a part of a body (in this embodiment, the thigh part TL and TR) and a third outer-side disposition portion 31B separable from and contactable with the third body-side disposition portion 31A and capable of changing a distance between the third body-side disposition portion 31A and the third outer-side disposition portion 31B. In this embodiment, the muscular strength assisting apparatus 1 may have two third assistance units 30 disposed on left and right sides, respectively, and each of the third assistance units 30 on the left and right sides may have the third actuator 31. Each of the third actuators 31 may have the third body-side disposition portion 31A, the third outer-side disposition portion 31B, and a tubular portion 31C formed of an elastic member joining the third body-side disposition portion 31A to the third outer-side disposition portion 31B.

The third actuator 31 may be a fluid pouring type actuator (a so-called artificial muscle type actuator) and have a configuration similar to that of the above-mentioned first actuator 11. That is, the third actuator 31 may switch between a state where a fluid flows into an inside of the tubular portion 31C and a state where the fluid is discharged therefrom so that the tubular portion 31C is elastically deformed, thus being capable of changing a distance between the third body-side disposition portion 31A and the third outer-side disposition portion 31B. As the above-mentioned fluid caused to flow into the inside of the tubular portion 31C, similarly to cases of the first actuator 11 and the second actuator 21, air may be used, which may be supplied from the air supply portion 41 in the control unit 40.

As shown in the figures, the third body-side disposition portion 31A of each of the two third actuators 31 may be connected to a thigh attachment tool 33. The thigh attachment tool 33 may have a left thigh attachment tool 33L attached to a left thigh TL of a human and a right thigh attachment tool 33R attached to a right thigh TR of the human (see Fig. 2). The third body-side disposition portion 31A of the third actuator 31 of one of the third assistance units 30 that is disposed on the left side may be connected to the left thigh attachment tool 33L via a wire 32A (see Fig. 2). The third body-side disposition portion 31A of the third actuator 31 of the other of the third assistance units 30 that is disposed on the right side may be connected to the right thigh attachment tool 33R via the wire 32A (see Fig. 2). Thus, in the third assistance units 30, the respective third body-side disposition portions 31A of the third actuators 31 may be indirectly mounted to the thighs TL and TR of a lower body of the human, respectively. The wire 32A may be a metallic wire or the like and made of a material having a high elastic modulus. Furthermore, the left thigh attachment tool 33L and the right thigh attachment tool 33R may be made of, for example, cloth and wrapped around the thighs TL and TR, respectively.

In this embodiment, an end portion of a left-side one of the wires 32A may be connected to the left thigh attachment tool 33L swingably in the up-down direction and the left-right direction, and an end portion of a right-side one of the wires 32A may be connected to the right thigh attachment tool 33R swingably in the up-down direction and the left-right direction. As a way to realize such a connection mode, for example, openable hooks (such as carabiners) provided at the end portions of the wires 32A on the left and right sides may be mounted to ring-shaped members provided in the left thigh attachment tool 33L and the right thigh attachment tool 33R, respectively.

Furthermore, in this embodiment, a leg posture detection sensor 34 may be provided in each of the above-mentioned left thigh attachment tool 33L and the right thigh attachment tool 33R, and the leg posture detection sensor 34 may output, to the control unit 40, a signal indicating a degree of bending when a human shifts from a posture in which the human stretches his/her leg part (a stretching posture) to a bending posture. As the leg posture detection sensor 34, for example, an electromyography sensor, an angle sensor, an acceleration sensor, a strain gauge, or the like can be used

On the other hand, the third outer-side disposition portion 31B of each of the two third actuators 31 may be mounted to the second assistance unit 20 via a wire 32B (see Fig. 1 or Fig. 2). In more detail, each of the third outer-side disposition portions 31B may be connected to the second outer-side disposition portion 21B in the second actuator 21 of the second assistance unit 20 via the wire 32B. Thus, in the third assistance units 30, the respective third outer-side disposition portions 31B of the third actuators 31 may be indirectly mounted to the second assistance unit 20. Similarly to the wire 32A, the wire 32B may be a metallic wire or the like and made of a material having a high elastic modulus. Furthermore, in this embodiment, an end portion of the wire 32B of each of the third assistance units 30 may be connected to a lower portion of the second outer-side disposition portion 21B of the second assistance unit 20 swingably in the up-down direction and the left-right direction. As a way to realize such a connection mode, for example, an openable hook (such as a carabiner) provided at the end portion of the wire 32B may be mounted to a ring-shaped member provided in the second outer-side disposition portion 21B.

Furthermore, in this embodiment, in a state where the third body-side disposition portion 31A of a left-side one of the third actuators 31 is connected to the left thigh TL via the left thigh attachment tool 33L, and the third body-side disposition portion 31A of a right-side one of the third actuators 31 is connected to the right thigh TR via the right thigh attachment tool 33R, on the back part B of a human, the two third assistance units 30 may be in a state of extending from the second assistance unit 20 to the left and right thighs TL and TR of the human by passing through a rear side of a hip part of the human. That is, in this embodiment, the third assistance units 30 may be mounted to the left and right thighs TL and TR, respectively, so that a direction connecting the second body-side disposition portion 21A to the second outer-side disposition portion 21B of the second actuator 21 of the second assistance unit 20 is non-parallel to a direction connecting the third body-side disposition portion 31A to the third outer-side disposition portion 31B of the third actuator 31 of each of the third assistance units 30. In this mounted state, preferably, in a case where a human having the muscular strength assisting apparatus 1 attached thereto is in a posture in which the human stretches his/her leg part, the third assistance units 30 may be adjusted to extend, while sagging, from the second assistance unit 20 to the left and right thighs TL and TR of the human, respectively. In this case, the human can effortlessly take a bending posture.

Furthermore, in a state where, as mentioned above, the end portion of the wire 32B of each of the third assistance units 30 is connected to the lower portion of the second outer-side disposition portion 21B of the second assistance unit 20, it can also be said that the third outer-side disposition portion 318 of the third actuator 31 of each of the third assistance units 30 is indirectly mounted to the lower back part W of a human via the wire 32B and the second assistance unit 20 mounted to the lower back part W. Accordingly, the second assistance unit 20 may function as a mounting member for indirectly mounting the third outer-side disposition portion 31B of the third actuator 31 of each of the third assistance units 30 to the lower back part W. At a position on the rear side of the lower back part W, the second assistance unit 20 functioning as the mounting member may connect the second outer-side disposition portion 21B thereof to the third outer-side disposition portion 31B of the third actuator 31 via the wire 32B, thus indirectly mounting the third outer-side disposition portion 31B of the third actuator 31 to the lower back part W.

In each of the third assistance units 30 described above, in a state before air from the air supply portion 41 in the control unit 40 is caused to flow into the above-mentioned tubular portion 31C in the third actuator 31, a distance between the third body-side disposition portion 31A and the third outer-side disposition portion 31B may be defined as a reference length. From this reference length state, air may be caused to flow into the inside of the tubular portion 31C, thus elastically deforming the tubular portion 31C so that the tubular portion 31C is radially inflated, and thus a length of the tubular portion 31C in an axial direction may be contracted. Thus, a distance between the third body-side disposition portion 31A and the third outer-side disposition portion 31B may become shorter than the reference length, so that a length of each of the third assistance units 30 as a whole may become shorter than a length thereof as a whole before the air is caused to flow into the tubular portion 31C. A length of each of the third assistance units 30 as a whole may refer to a length between each of the left and right thigh attachment tools 33L and 33R and the end portion of a corresponding one of the wires 32B on the left and right sides, which is connected to the second assistance unit 20.

On the other hand, when the above-mentioned air caused to flow into the inside of the tubular portion 31C is discharged, the radially directed inflation of the tubular portion 31C may be released, so that a length of the tubular portion 31C in the axial direction may recover to a state before the inflation. Thus, a distance between the third body-side disposition portion 31A and the third outer-side disposition portion 31B may recover to the reference length, so that a length of each of the third assistance units 30 as a whole may recover to a length thereof as a whole before the air is caused to flow into the tubular portion 31C.

Furthermore, also in this embodiment, when air is caused to flow into the inside of the above-mentioned tubular portion 31C so that a length of the tubular portion 31C in the axial direction is contracted, each of the third actuators 31 can be stopped from operating by, for example, restricting air inflow into and air discharge from the tubular portion 31C. Thus, in each of the third assistance units 30, in a state where the third actuator 31 is stopped from operating, a distance between the third body-side disposition portion 31A and the third outer-side disposition portion 31B of said third actuator 31 can be maintained at a predetermined length. Thus, in a state where the third actuator 31 is stopped from operating, each of the third assistance units 30 may be capable of maintaining a length thereof as a whole at a fixed value.

Furthermore, though not shown, a length adjustment mechanism for adjusting a length of the wire 32A may be provided between the wire 32A and the thigh attachment tool 33. Thus, a length of the wire 32A extending between the third body-side disposition portion 31A of the third actuator 31 and the thigh attachment tool 33 may be adjustable, and thus the wire 32A can be adjusted to an appropriate length depending on a size of a human body. In a case where a length of the wire 32A is adjusted by the length adjustment mechanism, the wire 32A may be maintained at the length thus adjusted Thus, even when pulled by the third body-side disposition portion 31A of the third actuator 31, the wire 32A may be prevented from having a length different from an adjusted length. It may also be possible that the length adjustment mechanism described above is provided at the wire 32B.

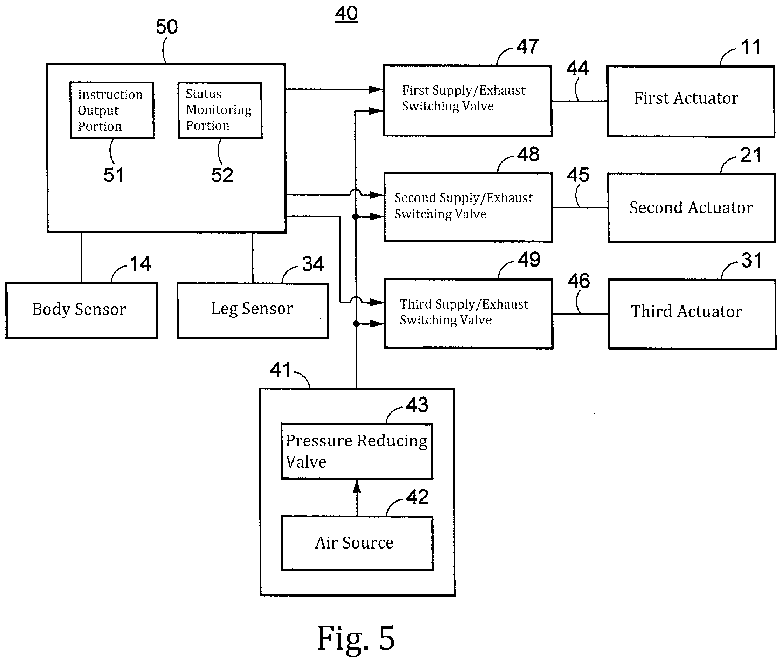

(Control Unit) Next, Fig. 1 shows the control unit 40. For the sake of convenience of description, a depiction of the control unit 40 is omitted in each of Fig. 2 to Fig. 4. Furthermore, Fig. 5 is a schematic view of a system configuration showing an electrical or mechanical connection mode between the control unit 40 and each of the assistance units 10, 20, and 30. As shown in Fig. 1 and Fig. 5, the control unit 40 of this embodiment may have the above-mentioned air supply portion 41 and a controller 50.

As shown in Fig.1 and Fig. 5, the air supply portion 41 may have an air source 42, a pressure reducing valve 43 connected to the air source 42, a first pipe 44 connecting the pressure reducing valve 43 to the first actuator 11, a second pipe 45 connecting the pressure reducing valve 43 to the second actuator 21, and two third pipes 46 each connecting the pressure reducing valve 43 to a corresponding one of the two third actuators 31. The first pipe 44 may be provided with a first supply/exhaust switching valve 47. The second pipe 45 may be provided with a second supply/exhaust switching valve 48. Each of the two third pipes 46 may be provided with a third supply/exhaust switching valve 49. One of the two third pipes 46 may be connected to the left-side one of the third actuators 31, and the other of the two third pipes 46 may be connected to the right-side one of the third actuators 31. The third pipes 46 and the third supply/exhaust switching valves 49 may be identical in configuration on the left and right sides, and Fig.1 and Fig. 5, therefore, show only one pair of one of the third pipes 46 and a corresponding one of the third supply/exhaust switching valves 49.

In the air supply portion 41, the pressure reducing valve 43 may reduce a pressure of compressed air in the air source 42 formed of, for example, a tank filled with compressed air to a predetermined value and supply the thus pressure-reduced compressed air to the first pipe 44, the second pipe 45, and the third pipes 46. The pressure reducing valve 43 may be capable of adjusting a pressure in the air source 42 to a desired value. This may enable adjustment of an operation speed of each of the actuators 11, 21, and 31 supplied with air via the pipes 44 to 46, respectively.

The first pipe 44 may be formed of a flexible tube or the like, and the first supply/exhaust switching valve 47 may be electrically connected to the controller 50. In accordance with an instruction from the controller 50, the first supply/exhaust switching valve 47 may switch, for example, between a state where air from the pressure reducing valve 43 is caused to flow into the inside of the tubular portion 11C of the first actuator 11 and a state where the air caused to flow into the inside of the tubular portion 11C is discharged

As the first supply/exhaust switching valve 47, there may be used, for example, a three-port valve having a connection port connected to the pressure reducing valve 43 via an upstream side of the first pipe 44, a supply port connected to the first actuator 11 via a downstream side of the first pipe 44, and a discharge port open to the air. In this case, in the first supply/exhaust switching valve 47, the connection port and the supply port may be formed to communicate with each other, and thus air from the pressure reducing valve 43 can be caused to flow into the inside of the tubular portion 11C of the first actuator 11. Furthermore, the supply port and the discharge port may be formed to communicate with each other, and thus the air caused to flow into the inside of the tubular portion 11C can be discharged Furthermore, after air has been caused to flow into the inside of the tubular portion 11C, all the ports may be blocked, so that the first actuator 11 can be stopped from operating, and a distance between the first body-side disposition portion 11A and the first outer-side disposition portion 11B of the first actuator 11 can also be maintained at a predetermined length.

Similarly, the second pipe 45 may be formed of a flexible tube or the like, and the second supply/exhaust switching valve 48 may be electrically connected to the controller 50. In accordance with an instruction from the controller 50, the second supply/exhaust switching valve 48 may switch, for example, between a state where air from the pressure reducing valve 43 is caused to flow into the inside of the bellows portion 21C of the second actuator 21 and a state where the air caused to flow into the inside of the bellows portion 21C is discharged.

As the second supply/exhaust switching valve 48, there may be used, for example, a three-port valve having a connection port connected to the pressure reducing valve 43 via an upstream side of the second pipe 45, a supply port connected to the second actuator 21 via a downstream side of the second pipe 45, and a discharge port open to the air. In this case, in the second supply/exhaust switching valve 48, the connection port and the supply port may be formed to communicate with each other, and thus air from the pressure reducing valve 43 can be caused to flow into the inside of the bellows portion 21C of the second actuator 21. Furthermore, the supply port and the discharge port may be formed to communicate with each other, and thus the air caused to flow into the inside of the bellows portion 21C can be discharged. Furthermore, after air has been caused to flow into the inside of the bellows portion 21C, all the ports may be blocked, so that the second actuator 21 can be stopped from operating, and a distance between the second body-side disposition portion 21A and the second outer-side disposition portion 21B of the second actuator 21 can also be maintained at a predetermined length.

Similarly, each of the third pipes 46 may be formed of a flexible tube or the like, and the third supply/exhaust switching valve 49 may be electrically connected to the controller 50. In accordance with an instruction from the controller 50, the third supply/exhaust switching valve 49 may switch, for example, between a state where air from the pressure reducing valve 43 is caused to flow into the inside of the tubular portion 31C of the third actuator 31 and a state where the air caused to flow into the inside of the tubular portion 31C is discharged

As the third supply/exhaust switching valve 49, there may be used, for example, a three-port valve having a connection port connected to the pressure reducing valve 43 via an upstream side of each of the third pipes 46, a supply port connected to the third actuator 31 via a downstream side of the each of the third pipes 46, and a discharge port open to the air. In this case, in the third supply/exhaust switching valve 49, the connection port and the supply port may be formed to communicate with each other, and thus air from the pressure reducing valve 43 can be caused to flow into the inside of the tubular portion 31C of the third actuator 31. Furthermore, the supply port and the discharge port may be formed to communicate with each other, and thus the air caused to flow into the inside of the tubular portion 31C can be discharged Furthermore, after air has been caused to flow into the inside of the tubular portion 31C, all the ports may be blocked, so that the third actuator 31 can be stopped from operating, and a distance between the third body-side disposition portion 31A and the third outer-side disposition portion 31B of the third actuator 31 can also be maintained at a predetermined length.

On the other hand, the controller 50 may be electrically connected to the above-mentioned supply/exhaust switching valves 47 to 49 and electrically connected also to the body posture detection sensor 14 and the leg posture detection sensor 34 mentioned above. In this embodiment, based on signals from the body posture detection sensor 14 and the leg posture detection sensor 34, the controller 50 may detect that a human has taken a forward bending posture and a leg bending posture. At this time, the controller 50 may output instructions to the supply/exhaust switching valves 47 to 49, thus controlling operations of the assistance units 10, 20, and 30.

As shown in Fig. 5, the controller 50 of this embodiment may have an instruction output portion 51 that outputs an instruction to each of the supply/exhaust switching valves 47 to 49 and a status monitoring portion 52. The status monitoring portion 52 of this embodiment may be a timer and monitor, for example, a lapse of time from an output of an instruction from the instruction output portion 51. Furthermore, in this embodiment, the controller 50 may output similar instructions, at the same timing, to the two third supply/exhaust switching valves 49 corresponding to the third actuators 31 on the left and right sides, respectively. It may also be possible that the controller 50 described above is formed of a CPU, a ROM, a RAM and so on or configured by including a plurality of logic circuits.

(Operation of Muscular Strength Assisting Apparatus) Next, a description is given of one example of an operation of the muscular strength assisting apparatus 1 of this embodiment configured as above.

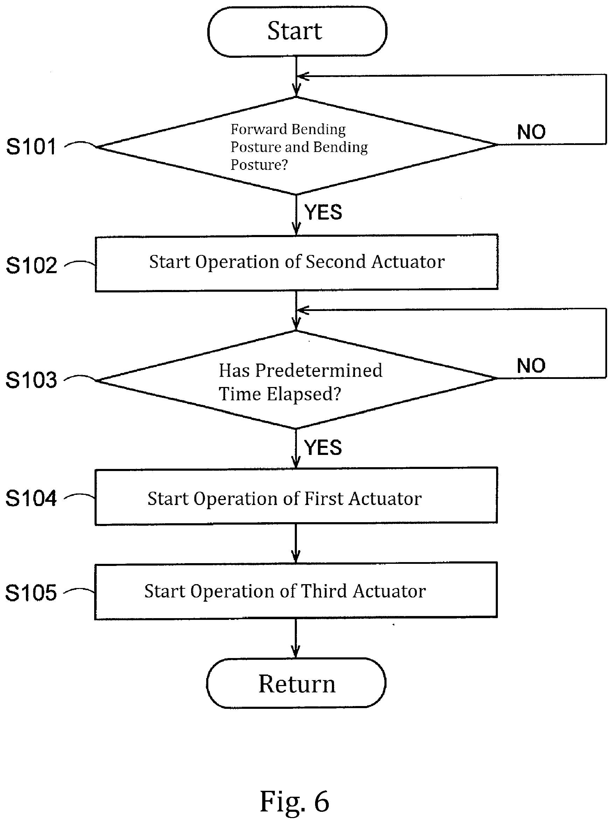

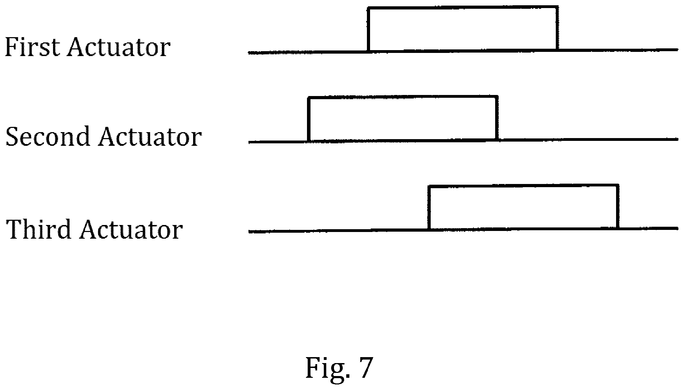

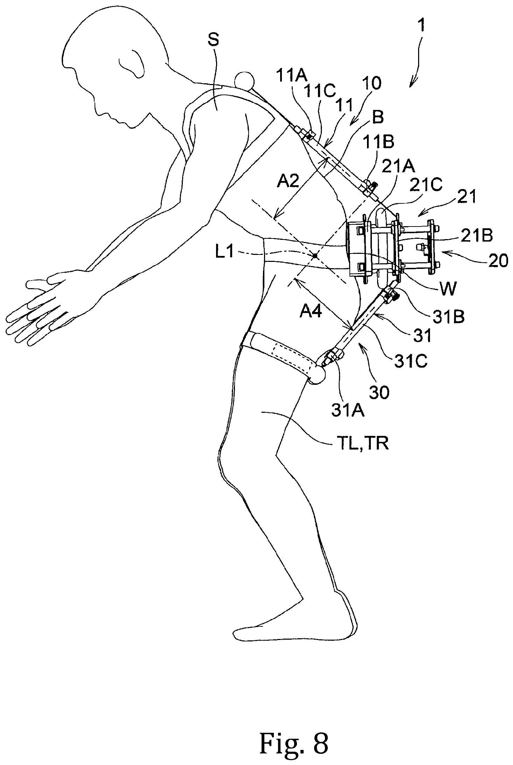

Fig. 6 shows a flow chart explaining one example of an operation of the muscular strength assisting apparatus 1 in this embodiment. Fig. 7 shows a timing chart corresponding to the operation explained in Fig. 6. Furthermore, Fig. 8 to Fig. 11 show respective working states of the assistance units 10, 20, and 30 of the muscular strength assisting apparatus 1 operating in accordance with the flow chart of Fig. 6. In brief outline, the muscular strength assisting apparatus 1 of this embodiment may control the assistance units 10,20, and 30 to assist a human in an activity of recovering to an upright posture and a leg stretching posture after taking a forward bending posture and a leg bending posture as shown in Fig. 8. By using Fig. 6 to Fig. 11, the following describes one example of the operation of the muscular strength assisting apparatus 1.

The muscular strength assisting apparatus 1 according to this embodiment may be attached to a human, and then an unshown activation switch thereof may be operated to bring the muscular strength assisting apparatus 1 into an activated state. At this time, first, as shown in step S101 in Fig. 6, based on signals from the body posture detection sensor 14 and the leg posture detection sensor 34, the controller 50 may monitor whether or not the human has taken a forward bending posture and a leg bending posture. In a case where, based on the body posture detection sensor 14 and the leg posture detection sensor 34, it is determined that the human has taken a forward bending posture and a leg bending posture as shown in Fig. 8 (in a case of YES), a process may be advanced to step S102. On the other hand, in a case where it is not determined that the human has taken a forward bending posture and a leg bending posture (in a case of NO), monitoring is continuously performed in step S101.

As shown in Fig. 8, in the muscular strength assisting apparatus 1 according to this embodiment, when the human takes a forward bending posture and a leg bending posture, the first assistance unit 10 and the third assistance units 30 may be pulled forward. In this case, when the first assistance unit 10 has previously been adjusted to extend, while sagging, from the second assistance unit 20 to the shoulder part S of the human in an upright posture, the sag of the first assistance unit 10 may be eliminated At this time, in a case where the sag of the first assistance unit 10 between the second assistance unit 20 and the shoulder part S is completely eliminated the bellows portion 21C of the second actuator 21 may be compressed Furthermore, when the third assistance units 30 have previously been adjusted to extend, while sagging, from the second assistance unit 20 to the thigh part TL and TR of the human in a leg stretching posture, the sag of the third assistance units 30 may be eliminated At this time, in a case where the sag of the third assistance units 30 between the second assistance unit 20 and the thigh part TL and TR is completely eliminated the bellows portion 21C of the second actuator 21 may be compressed As shown in Fig. 8, in this example, the bellows portion 21C may be in a compressed state.

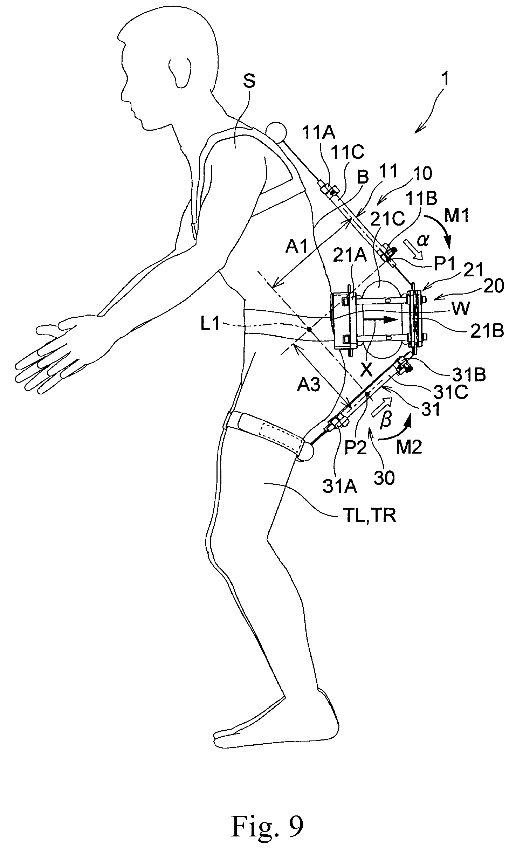

In step S102 after determination that a forward bending posture and a leg bending posture have been taken, at predetermined timing (timing such as, for example, several seconds later from the determination or when an input is made by the human), the controller 50 may control the instruction output portion 51 to output an instruction to the second supply/exhaust switching valve 48 so as to bring about a state where air from the pressure reducing valve 43 is caused to flow into the inside of the bellows portion 21C of the second actuator 21. Thus, as shown in Fig. 9, the second actuator 21 may be operated so that, as shown by an arrow X in the figure, a distance between the second body-side disposition portion 21A and the second outer-side disposition portion 21B of the second actuator 21 becomes longer than the reference length before the air is caused to flow into the bellows portion 21C.

As shown in Fig. 9, in a state where a distance between the second body-side disposition portion 21A and the second outer-side disposition portion 21B of the second actuator 21 has become longer, the first assistance unit 10 and the third assistance units 30 may be pulled rearward This may cause the human to shift his/her posture so that the shoulder part S rises from the originally taken forward bending posture and the thigh part TL and TR rise from the originally taken leg bending posture. Herein, in this example, after a predetermined amount of air has been caused to flow into the inside of the bellows portion 21C, the controller 50 may stop the second actuator 21 from operating, and thus a distance between the second body-side disposition portion 21A and the second outer-side disposition portion 21B of the second actuator 21 may be maintained at a predetermined length.

Next, in step S103, the controller 50 may monitor whether or not a predetermined time has elapsed from a start of an operation of the second actuator 21. In a case where a lapse of the predetermined time is detected (in a case of YES), the process may be advanced to step S104, and in a case where the predetermined time has not yet elapsed (in a case of NO), monitoring may be continuously performed at step S103.

Then, in step S104, the controller 50 may control the instruction output portion 51 to output an instruction to the first supply/exhaust switching valve 47 so as to bring about a state where air from the pressure reducing valve 43 is caused to flow into the inside of the tubular portion 11C of the first actuator 11. Thus, the first actuator 11 may be operated so that a distance between the first body-side disposition portion 11A and the first outer-side disposition portion 11B of the first actuator 11 becomes shorter than the reference length before the air is caused to flow into the tubular portion 11C, and thus a length of the first assistance unit 10 as a whole may become shorter than a length thereof as a whole before a fluid is caused to flow into the tubular portion 11C.

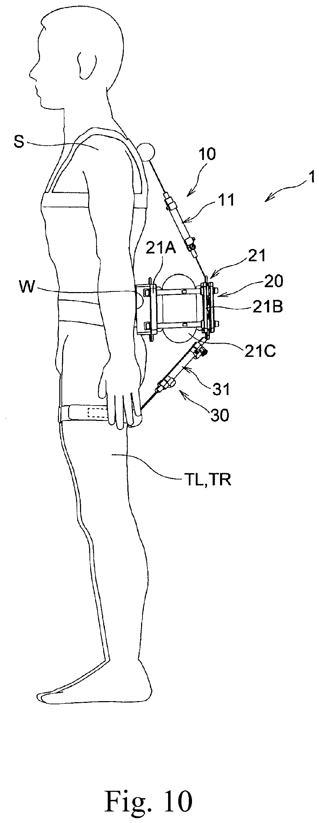

At this time, in the muscular strength assisting apparatus 1, a force indicated by an arrow α in Fig. 9, which is directed obliquely downward to the rear, may be generated in the first actuator 11, and with this force acting as a point of action, a moment M1 for assisting in an activity of moving the shoulder part S and the lower back part W relative to each other may be generated The moment M1, to be exact, may be a moment about a joint point, as a rotation axis L1, between the lower back part W and the back part B positioned between the lower back part W and the shoulder part S. For the moment M1, a moment arm A1 may be set between the rotation axis L1 and an intersection P1 between the first actuator 11 and a straight line extending from the rotation axis L1 and orthogonal to a direction connecting the first body-side disposition portion 11A to the first outer-side disposition portion 11B of the first actuator 11. Since the shoulder part S may be swivelably or bendably joined to the lower back part W via the back part B, in a case where the moment M1 described above acts on the shoulder part S, the human may be assisted in an activity of recovering from a forward bending posture to an upright posture. Thus, according to the muscular strength assisting apparatus 1, as shown in Fig. 10, the human can readily be caused to recover from a forward bending posture to an upright posture.

Furthermore, the above-mentioned moment arm A1 may be larger than a moment arm A2 shown in Fig. 8, which is a moment arm before the second actuator 21 increases a distance between the second body-side disposition portion 21A and the second outer-side disposition portion 21B thereof. Thus, in this embodiment, in a state after the increase in moment arm from the moment arm A2 to the moment arm A1, even in a case where an output of the first actuator 11 is kept lower than would be required for obtaining a moment of a desired magnitude in a state of having the moment arm A2, the moment M1 equal in magnitude to the above-mentioned moment of a desired magnitude can be ensured Thus, according to this embodiment, a size reduction of the first actuator 11 can be achieved

Furthermore, in this example, after a predetermined amount of air has been caused to flow into the inside of the tubular portion 11C, the controller 50 may stop the first actuator 11 from operating, and thus a distance between the first body-side disposition portion 11A and the first outer-side disposition portion 11B of the first actuator 11 may be maintained at a predetermined length. In this case, for example, when the human, while supporting a heavy item, recovers from a forward bending posture to an upright posture, a body of the human may be prevented from moving to a forward bending posture side under an influence of the heavy item, and thus the human can stably recover to the upright posture. Furthermore, the above-mentioned monitoring of the predetermined time performed by the status monitoring portion 52 in step S103 may be a process step of determining timing for operating the first actuator 11, which may be useful in the following sense. That is, in a configuration in which, as in this embodiment, it is monitored whether or not a predetermined time has elapsed from a start of an operation of the second actuator 21, the timing for operating the first actuator 11 can be determined without using a special sensor.

Furthermore, in subsequent step S105, the controller 50 may control the instruction output portion 51 to output an instruction to the third supply/exhaust switching valve 49 so as to bring about a state where air from the pressure reducing valve 43 is caused to flow into the inside of the tubular portion 31C of the third actuator 31. Thus, the third actuator 31 may be operated so that a distance between the third body-side disposition portion 31A and the third outer-side disposition portion 31B of the third actuator 31 becomes shorter than the reference length before the air is caused to flow into the tubular portion 31C, and thus a length of each of the third assistance units 30 as a whole may become shorter than a length thereof as a whole before a fluid is caused to flow into the tubular portion 31C.

At this time, in the muscular strength assisting apparatus 1, a force indicated by an arrow β in Fig. 9, which is directed obliquely upward to the rear, may be generated in the third actuator 31, and with this force acting as a point of action, a moment M2 for assisting in an activity of moving the thigh part TL and TR and the lower back part W relative to each other may be generated The moment M2, to be exact, may be a moment about a joint point, as the rotation axis L1, between the lower back part W and the thigh part TL and TR. For the moment M2, a moment arm A3 may be set between the rotation axis L1 and an intersection P2 between the third actuator 31 and a straight line extending from the rotation axis L1 and orthogonal to a direction connecting the third body-side disposition portion 31A to the third outer-side disposition portion 31B of the third actuator 31. In a case where the moment M2 described above acts on the thigh parts TL and TR, the human may be assisted in an activity of recovering from a leg bending posture to a stretching posture. Thus, according to the muscular strength assisting apparatus 1, as shown in Fig. 10, the human can readily be caused to recover from a bending posture to a stretching posture.

Furthermore, the above-mentioned moment arm A3 may be larger than a moment arm A4 shown in Fig. 8, which is a moment arm before the second actuator 21 increases a distance between the second body-side disposition portion 21A and the second outer-side disposition portion 21B of the second actuator 21. Thus, in this embodiment, in a state after the increase in moment arm from the moment arm A4 to the moment arm A3, even in a case where an output of the third actuator 31 is kept lower than would be required for obtaining a moment of a desired magnitude in a state of having the moment arm A4, the moment M2 equal in magnitude to the above-mentioned moment of a desired magnitude can be ensured Thus, according to this embodiment, a size reduction of the third actuator 31 can be achieved.

Furthermore, in this example, after a predetermined amount of air has been caused to flow into the inside of the tubular portion 31C, the controller 50 may stop the third actuator 31 from operating, and thus a distance between the third body-side disposition portion 31A and the third outer-side disposition portion 31B of the third actuator 31 may be maintained at a predetermined length. In this case, for example, when the human, while supporting a heavy item, recovers from a bending posture to a stretching posture, a body of the human may be prevented from moving to a bending posture side under an influence of the heavy item, and thus the human can stably recover to the stretching posture.

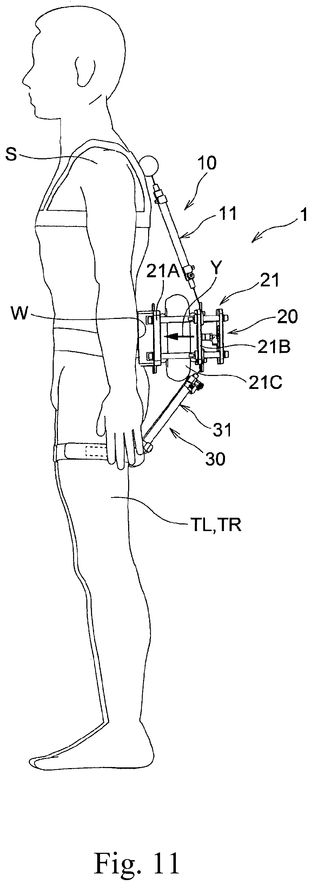

After that, in this embodiment, as is apparent from a comparison between Fig. 10 and Fig. 11, a distance between the second body-side disposition portion 21A and the second outer-side disposition portion 21B of the second actuator 21 may recover, as shown by an arrow Y in Fig. 11, to the reference length before the air is caused to flow into the bellows portion 21C. That is, the controller 50 may output an instruction to the second supply/exhaust switching valve 48 so as to bring about a state where the air caused to flow into the inside of the bellows portion 21C is discharged This may suppress an undesirable load applied on the human by the second actuator 21. Furthermore, at this time, a length of each of the first assistance unit 10 and the third assistance units 30 as a whole may also be brought into an expanded state as a result of the air caused to flow into the inside of the above-mentioned tubular portion 11C or 31C being discharged This may suppress an undesirable bad applied on the human by the first actuator 11 and the third actuator 31.

As mentioned above, in this embodiment, when a human has taken a forward bending posture and a leg bending posture, first, the second actuator 21 may be operated, and then, the first actuator 11 may be operated, after which the third actuator 31 may be cause to operate. Thus, in this embodiment, in generating a moment for assisting in a body activity by operating the first actuator 11 and the third actuator 31, an increased moment arm may be reliably ensured Furthermore, after the second actuator 21 has been operated, before the third actuator 31 is operated, the first actuator 11 may be operated, and thus a bad applied on the lower back part W of a human can be effectively reduced That is, in a case where a forward bending posture is taken for a long time, a load applied on the lower back part W may be increased Furthermore, as shown in Fig. 7, in this embodiment, while the second actuator 21 is being expanded, the first actuator 11 may start to be contracted, and while the first actuator 11 is being contracted the third actuator 31 may start to be contracted In this embodiment, operational timings may be set to overlap with each other as described above, and thus a time required for recovering to an upright posture and a leg stretching posture after taking a forward bending posture and a leg bending posture can be favorably reduced

(Advantageous Effects) In the above-described muscular strength assisting apparatus 1 according to this embodiment, the first assistance unit 10 may be mounted to the shoulder part S and the second assistance unit 20 may be mounted to the lower back part W so that a direction connecting the second body-side disposition portion 21A to the second outer-side disposition portion 21B of the second actuator 21 of the second assistance unit 20 is non-parallel to a direction connecting the first body-side disposition portion 11A to the first outer-side disposition portion 11B of the first actuator 11 of the first assistance unit 10. In this state, the first actuator 11 may change a distance between the first body-side disposition portion 11A and the first outer-side disposition portion 11B. At this time, a force may be generated in the first actuator 11, and with the force acting as a point of action, a moment for assisting in an activity of moving the shoulder part S and the lower back part W relative to each other can be generated Specifically, in this embodiment, a distance between the first body-side disposition portion 11A and the first outer-side disposition portion 11B may be decreased, and thus a human can be assisted in an activity of recovering from a forward bending posture to an upright posture. Further, at this time, the second actuator 21 increases a distance between the second body-side disposition portion 21A and the second outer-side disposition portion of 21B of the second actuator 21 and thus can increase a moment arm of a moment. Thus, in a state after the moment arm has thus been increased, even in a case where an output of the first actuator 11 of the first assistance unit 10 is kept lower than would be required for obtaining a moment of a desired magnitude in a state before the moment arm is increased, a moment equal in magnitude to the above-mentioned moment of a desired magnitude can be ensured.

Thus, according to the muscular strength assisting apparatus 1 of this embodiment, it is possible to ensure a moment of a desired magnitude for assisting in an activity of moving the shoulder part S and the lower back part W relative to each other, while keeping an output of the first actuator 11 low, the first actuator 11 generating a force at a point of action of said moment, and thus to suppress a size increase and a weight increase of the apparatus as a whole.

Furthermore, in the muscular strength assisting apparatus 1 according to this embodiment, the third assistance units 30 may be mounted to the thigh part TL and TR and the second assistance unit 20 may be mounted to the lower back part W so that a direction connecting the second body-side disposition portion 21A to the second outer-side disposition portion 21B of the second actuator 21 of the second assistance unit 20 is non-parallel to a direction connecting the third body-side disposition portion 31A to the third outer-side disposition portion 31B of the third actuator 31 of each of the third assistance units 30. In this state, the third actuator 31 may change a distance between the third body-side disposition portion 31A and the third outer-side disposition portion 31B. At this time, a force may be generated in the third actuator 31, and with the force acting as a point of action, a moment for assisting in an activity of moving the thigh part TL and TR and the lower back part W relative to each other can be generated Specifically, in this embodiment, a distance between the third body-side disposition portion 31A and the third outer-side disposition portion 31B may be decreased, and thus a human can be assisted in an activity of recovering from a bending posture in which the human bends his/her legs to a stretching posture. Further, at this time, the second actuator 21 increases a distance between the second body-side disposition portion 21A and the second outer-side disposition portion of 21B of the second actuator 21 and thus can increase a moment arm of a moment. Thus, in a state after the moment arm has thus been increased, even in a case where an output of the third actuator 31 of each of the third assistance units 30 is kept lower than would be required for obtaining a moment of a desired magnitude in a state before the moment arm is increased, a moment equal in magnitude to the above-mentioned moment of a desired magnitude can be ensured

Thus, according to the muscular strength assisting apparatus 1 of this embodiment, it is possible to ensure a moment of a desired magnitude for assisting in an activity of moving the thigh part TL and TR and the lower back part W relative to each other, while keeping an output of the third actuator 31 low, the third actuator 31 generating a force at a point of action of said moment, and thus to suppress a size increase and a weight increase of the apparatus as a whole.

Furthermore, in this embodiment, in a state where the second actuator 21 is stopped from operating, the second assistance unit 20 may be capable of maintaining a distance between the second body-side disposition portion 21A and the second outer-side disposition portion 21B of said second actuator 21 at a predetermined length. Thus, when a distance between the second body-side disposition portion 21A and the second outer-side disposition portion 21B is increased in order to ensure a moment arm, the moment arm can be maintained in an increased state, and thus a human can be stably assisted in his/her activities.

Furthermore, in this embodiment, in a state where the first actuator 11 is stopped from operating, the first assistance unit 10 may be capable of maintaining a distance between the first body-side disposition portion 11A and the first outer-side disposition portion 11B of said first actuator 11 at a predetermined length. Thus, when a moment is generated the first actuator 11 may be stopped from operating, and thus a distance between the first body-side disposition portion 11A and the first outer-side disposition portion 11B of the first actuator 11 can be maintained at a predetermined length. Thus, for example, when a human, while supporting a heavy item, recovers from a forward bending posture to an upright posture, a body of the human may be prevented from moving to a forward bending posture side under an influence of the heavy item, and thus the human can stably recover to the upright posture. Furthermore, even after that, a distance between the first body-side disposition portion 11A and the first outer-side disposition portion 11B of the first actuator 11 may be maintained at a predetermined length, so that assistance for maintaining the upright posture may be enabled Furthermore, when a human places a heavy item on a floor surface or the like, air may be controlled to be discharged from the tubular portion 11C so that a distance between the first body-side disposition portion 11A and the first outer-side disposition portion 11B of the first actuator 11 is gradually increased and thus a load applied on the lower back part W of the human can be reduced

Furthermore, in a state where the third actuator 31 is stopped from operating, each of the third assistance units 30 may be capable of maintaining a distance between the third body-side disposition portion 31A and the third outer-side disposition portion 31B of said third actuator 31 at a predetermined length. Thus, when a moment is generated, the third actuator 31 may be stopped from operating, and thus a distance between the third body-side disposition portion 31A and the third outer-side disposition portion 31B of the third actuator 31 can be maintained at a predetermined length. Thus, for example, when a human, while supporting a heavy item, recovers from a bending posture to a stretching posture, a body of the human may be prevented from moving to a bending posture side under an influence of the heavy item, and thus the human can stably recover to the stretching posture. Furthermore, even after that, a distance between the third body-side disposition portion 31A and the third outer-side disposition portion 31B of the third actuator 31 may be maintained at a predetermined length, so that assistance for maintaining a leg stretching posture may be enabled Furthermore, when a human places a heavy item on a floor surface or the like, air may be controlled to be discharged from the tubular portion 31C so that a distance between the third body-side disposition portion 31A and the third outer-side disposition portion 31B of the third actuator 31 is gradually increased, and thus a bad applied on the lower back part W of the human can be reduced

Furthermore, in this embodiment, before the first actuator 11 and the third actuator 31 are operated, the controller 50 may operate the second actuator 21 so that the second outer-side disposition portion 21B of the second actuator 21 moves away from the bwer back part W. Thus, an increased moment arm may be reliably ensured, so that at the time of output of the first actuator 11 and at the time of output of the third actuator 31, a moment having an increased moment arm can be reliably generated

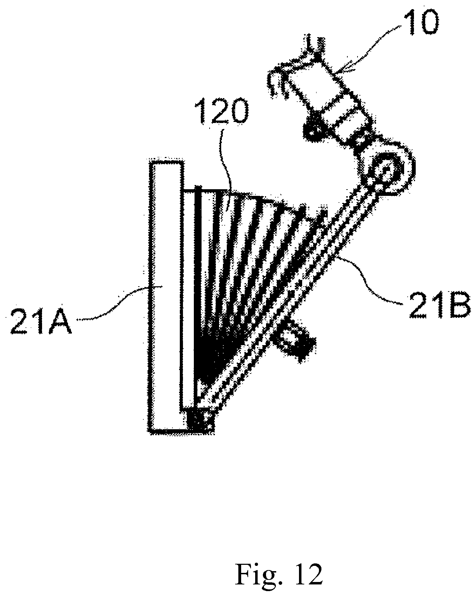

While the foregoing has described one embodiment of the present invention, the present invention may not be limited to the foregoing embodiment. By using Fig. 12 to Fig. 38, the following describes modification examples of one embodiment mentioned above. In the modification examples described below, components common to those in the foregoing embodiment are indicated by the same reference characters.

(Modification Examples of Second Actuator) The foregoing embodiment has described a configuration in which the second actuator 21 may have the second body-side disposition portion 21A, the second outer-side disposition portion 21B, and the bellows portion 21C formed of an elastic member joining the second body-side disposition portion 21A to the second outer-side disposition portion 21B, and the second actuator 21 may switch between a state where a fluid flows into the inside of the bellows portion 21C and a state where the fluid is discharged therefrom so that the bellows portion 21C is expanded/contracted thus changing a distance between the second body-side disposition portion 21A and the second outer-side disposition portion 21B. Fig. 12 to Fig. 22 show various modification examples of the second actuator 21.

A modification example shown in Fig. 12 may have a configuration in which a bellows portion 120 joining a second body-side disposition portion 21A to a second outer-side disposition portion 21B of a second actuator 21 is developed from a rectangular plate-shaped state in a circumferential direction about one side thereof as a rotation axis. The second outer-side disposition portion 21B joined to the bellows portion 120 may move in the circumferential direction about the above-described rotation axis as an axial center so as to be away from the second body-side disposition portion 21A. Fig.12 shows a developed state of the bellows portion 120. It may also be possible that the bellows portion 120 is driven by using compressed air from the air supply portion 41. Furthermore, in this example, the first outer-side disposition portion 11B of the first actuator 11 in the first assistance unit 10 may be mounted to the second outer-side disposition portion 21B without using a wire or the like. Furthermore, though not shown, the third assistance units 30 could be mounted to appropriate locations on the second outer-side disposition portion 21B, respectively.

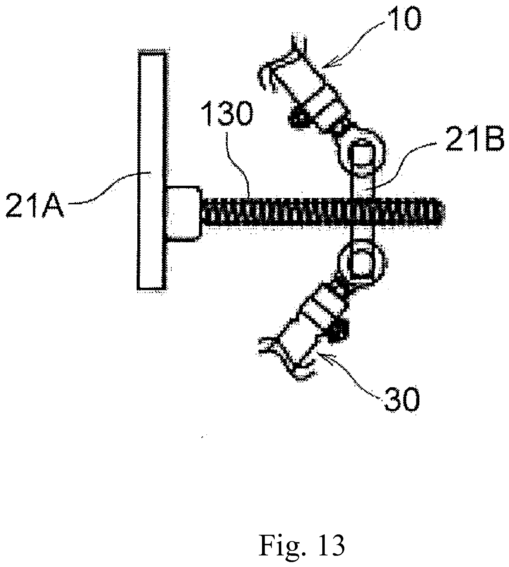

In a modification example shown in Fig. 13, a screw 130 may be provided on a second body-side disposition portion 21A of a second actuator 21, and a second outer-side disposition portion 21B may be screwed on the screw 130. In this configuration, the screw 130 may rotate to cause the second outer-side disposition portion 21B to move with respect to the second body-side disposition portion 21A. It may also be possible that the screw 130 is caused to rotate by an air motor or the like that rotates using compressed air from the air supply portion 41. Furthermore, in this example, the first outer-side disposition portion 11B of the first actuator 11 in the first assistance unit 10 and the third outer-side disposition portion 31B of the third actuator 31 in each of the third assistance units 30 may be mounted to the second outer-side disposition portion 21B without using a wire or the like.

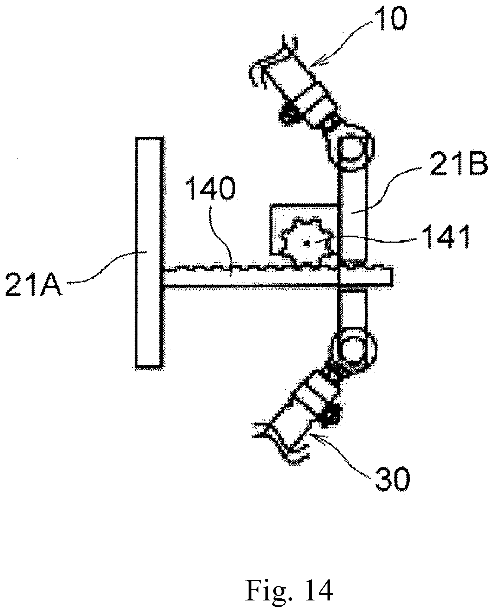

In a modification example shown in Fig. 14, a rack 140 may be provided on a second body-side disposition portion 21A of a second actuator 21, and a second outer-side disposition portion 21B of the second actuator 21B may be provided with a pinion gear 141 that meshes with the rack 14. In this configuration, the pinion gear 141 may rotate to cause the second outer-side disposition portion 21B to move with respect to the second body-side disposition portion 21A. It may also be possible that the pinion gear 141 is caused to rotate by an air motor or the like that rotates using compressed air from the air supply portion 41. Furthermore, in this example, the first outer-side disposition portion 11B of the first actuator 11 in the first assistance unit 10 and the third outer-side disposition portion 31B of the third actuator 31 in each of the third assistance units 30 may be mounted to the second outer-side disposition portion 21B without using a wire or the like.

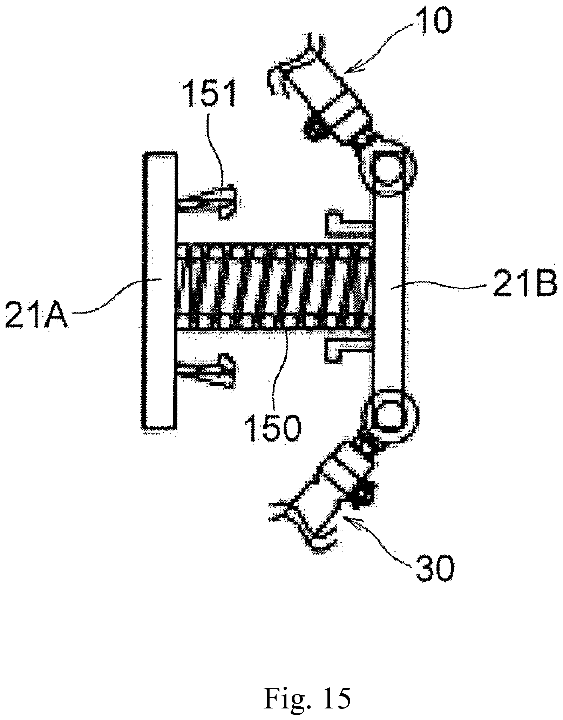

In a modification example shown in Fig. 15, a spring 150 may be provided between a second body-side disposition portion 21A and a second outer-side disposition portion 21B of a second actuator 21. The second body-side disposition portion 21A may be provided with an electromagnetic lock 151 having a claw that latches the spring 150 so as to retain the spring 150 in a compressed state. In this configuration, the spring 150 retained in a compressed state by the electromagnetic lock 151 may be released from the retained state, thus causing the second outer-side disposition portion 21B to move with respect to the second body-side disposition portion 21A. Furthermore, in this example, the first outer-side disposition portion 11B of the first actuator 11 in the first assistance unit 10 and the third outer-side disposition portion 31B of the third actuator 31 in each of the third assistance units 30 may be mounted to the second outer-side disposition portion 21B without using a wire or the like.

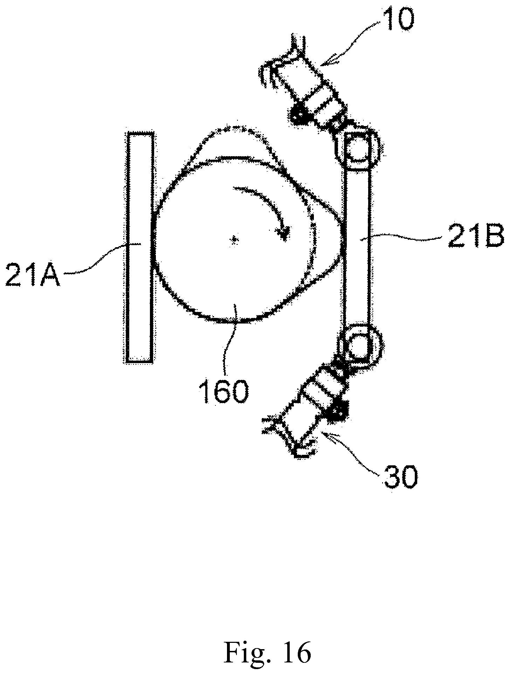

In a modification example shown in Fig. 16, a cam 160 may be rotatably provided between a second body-side disposition portion 21A and a second outer-side disposition portion 21B of a second actuator 21. In this configuration, when the cam160 rotates, a cam ridge thereof may push the second outer-side disposition portion 21B, thus causing the second outer-side disposition portion 21B to move with respect to the second body-side disposition portion 21A. It may also be possible that the cam 160 is caused to rotate by an air motor or the like that rotates using compressed air from the air supply portion 41. Furthermore, in this example, the first outer-side disposition portion 11B of the first actuator 11 in the first assistance unit 10 and the third outer-side disposition portion 31B of the third actuator 31 in each of the third assistance units 30 may be mounted to the second outer-side disposition portion 21B without using a wire or the like.

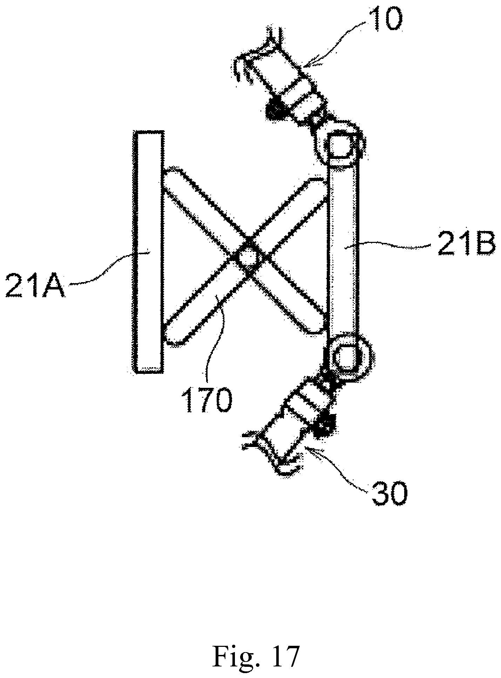

In a modification example shown in Fig. 17, an expandable/contractible link member 170 may be provided between a second body-side disposition portion 21A and a second outer-side disposition portion 21B of a second actuator 21. In this configuration, the link member 170 may be expanded/contracted to cause the second outer-side disposition portion 21B to move with respect to the second body-side disposition portion 21A. It may also be possible that the link member 170 is caused to rotate by an air motor or the like that rotates using compressed air from the air supply portion 41. Furthermore, in this example, the first outer-side disposition portion 11B of the first actuator 11 in the first assistance unit 10 and the third outer-side disposition portion 31B of the third actuator 31 in each of the third assistance units 30 may be mounted to the second outer-side disposition portion 21B without using a wire or the like.

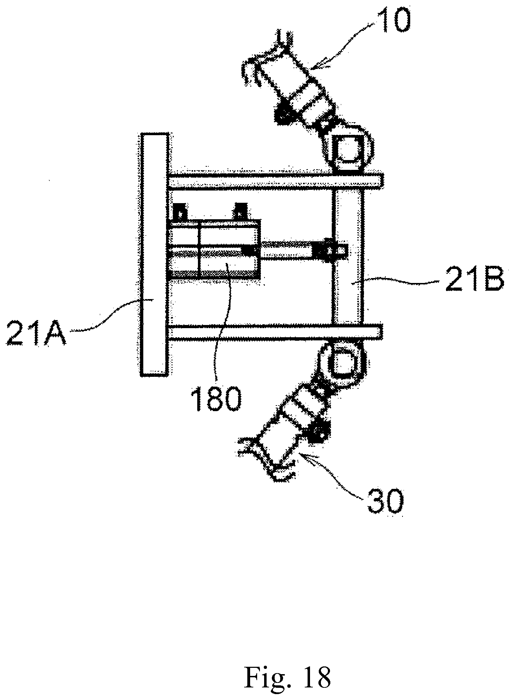

In a modification example shown in Fig.18, an air cylinder 180 may be provided between a second body-side disposition portion 21A and a second outer-side disposition portion 21B of a second actuator 21. In this configuration, a rod of the air cylinder 180 may be expanded/contracted to cause the second outer-side disposition portion 21B to move with respect to the second body-side disposition portion 21A. It may also be possible that the air cylinder 180 is driven by using compressed air from the air supply portion 41. Furthermore, in this example, the first outer-side disposition portion 11B of the first actuator 11 in the first assistance unit 10 and the third outer-side disposition portion 31B of the third actuator 31 in each of the third assistance units 30 may be mounted to the second outer-side disposition portion 21B without using a wire or the like.

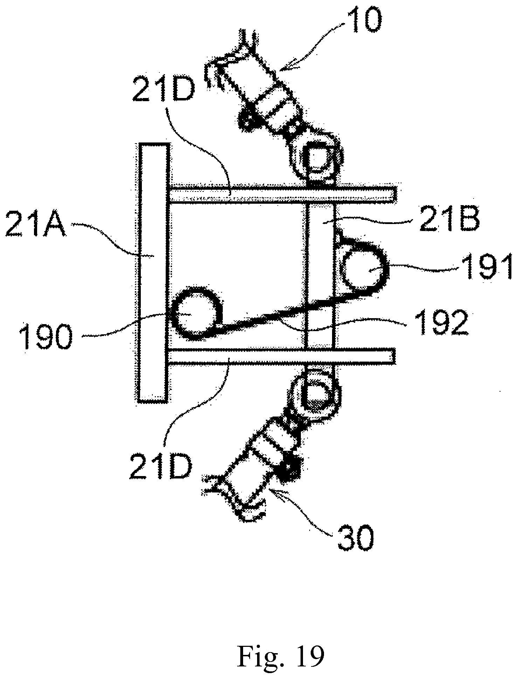

In a modification example shown in Fig. 19, a plurality of bar-shaped guide members 21D may be provided in an outer peripheral portion of a second body-side disposition portion 21A, and an outer peripheral portion of a second outer-side disposition portion 21B may be slidably inserted on the guide members 21D. In a second actuator 21, the second body-side disposition portion 21A may be provided with a first pulley 190, and the second outer-side disposition portion 21B may be provided with a second pulley 191, with a chain 192 wound over the pulleys 190 and 191. In this configuration, either one of the pulleys 190 and 191 may be selectively caused to rotate to wind the chain 192, thus causing the second outer-side disposition portion 21B to move with respect to the second body-side disposition portion 21A. It may also be possible that the pulleys 190 and 191 are caused to rotate by an air motor or the like that rotates using compressed air from the air supply portion 41. Furthermore, in this example, the first outer-side disposition portion 11B of the first actuator 11 in the first assistance unit 10 and the third outer-side disposition portion 31B of the third actuator 31 in each of the third assistance units 30 may be mounted to the second outer-side disposition portion 21B without using a wire or the like.

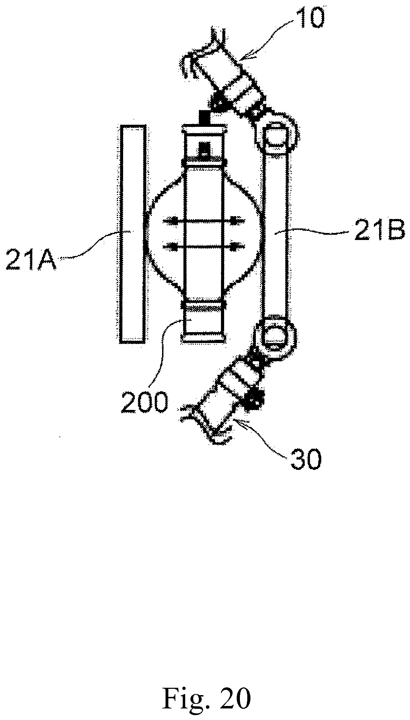

In a modification example shown in Fig. 20, a fluid pouring type actuator portion 20 (a so-called artificial muscle type actuator) may be provided between a second body-side disposition portion 21A and a second outer-side disposition portion 21B of a second actuator 21. In this configuration, a fluid may be caused to flow into the fluid pouring type actuator portion 200 so that a tubular portion thereof is radially inflated thus causing the second outer-side disposition portion 21B to move with respect to the second body-side disposition portion 21A. It may also be possible that the fluid pouring type actuator portion 200 is driven by using compressed air from the air supply portion 41. Furthermore, in this example, the first outer-side disposition portion 11B of the first actuator 11 in the first assistance unit 10 and the third outer-side disposition portion 31B of the third actuator 31 in each of the third assistance units 30 may be mounted to the second outer-side disposition portion 21B without using a wire or the like.

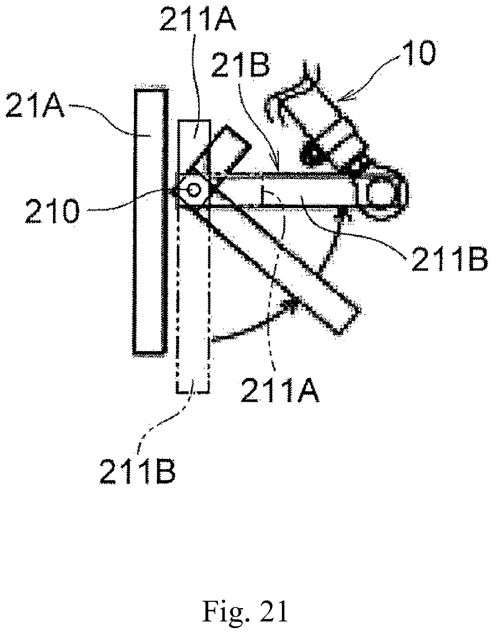

In a modification example shown in Fig. 21, a second body-side disposition portion 21A of a second actuator 21 may be provided with a rotary shaft 210, and a second outer-side disposition portion 21B may be rotatably supported to the rotary shaft 210. The second outer-side disposition portion 21B may have a first side portion 211A and a second side portion 211B, and the first side portion 211A and the second side portion 211B may be coupled to each other so as to form an L-shape (a crank shape). In the second outer-side disposition portion 21B, a coupling portion between the first side portion 211A and the second side portion 211B may be supported to the rotary shaft 210. In this example, the first outer-side disposition portion 11B of the first actuator 11 may be mounted to a distal end of the second side portion 211B as one of the first side portion 211A and the second side portion 211B without using a wire or the like. Further, in this configuration, from a state where the first side portion 211A extends toward a rear side and the second side portion 211B extends downward (a state shown by a chain double-dashed line), the second outer-side disposition portion 21B may be caused to rotate about the rotary shaft 210 so that the second side portion 211B rotationally moves upward, and thus a distal end of the second side portion 211B of the second ouster-side disposition portion 21B may move away from the second body-side disposition portion 21A. It may also be possible that the second out-side disposition portion 21B is caused to rotate by an air motor or the like that rotates using compressed air from the air supply portion 41.

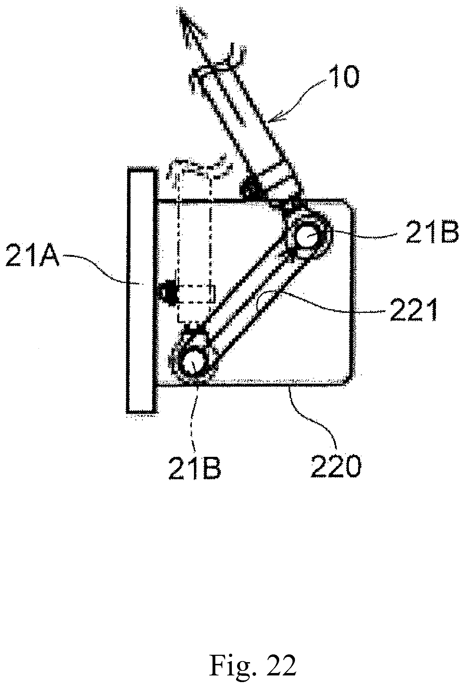

In a modification example shown in Fig. 22, a second body-side disposition portion 21A of a second actuator 21 may be provided with a guiding member 220 extending rearward, and a guiding groove 221 extending obliquely upward to the rear may be formed in the guiding member 220. Further, a second outer-side disposition portion 21B may be a shaft member and inserted in the guiding groove 221. In this configuration, the second outer-side disposition portion 21B may move obliquely upward to the rear along the guiding groove 221, and therefore, the second outer-side disposition portion 21B may move with respect to the second body-side disposition portion 21A.

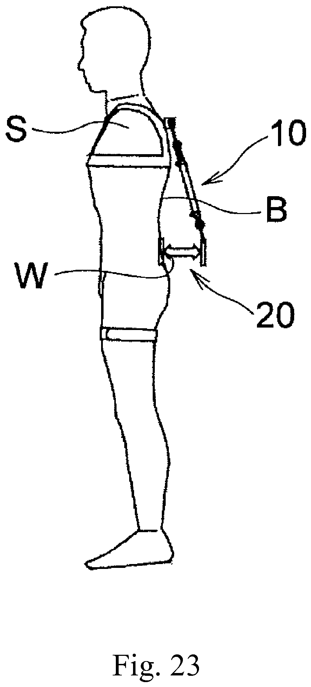

(Modification Examples of Mounting Positions of Assistance Units) The muscular strength assisting apparatus 1 described in the foregoing embodiment may have the first assistance unit 10, the second assistance unit 20, and the third assistance unit 30. In this configuration, the first assistance unit 10 may be mounted to the shoulder part S, the second assistance unit 20 may be mounted to the lower back part W, and the third assistance unit 30 may be mounted to the thigh part TL and TR. Fig. 23 to Fig. 35 show various modification examples of mounting positions of the assistance units.

In a modification example shown in Fig. 23, a muscular strength assisting apparatus 1 may have a first assistance unit 10 and a second assistance unit 20, and the third assistance unit 30 may not be provided therein. The first assistance unit 10 may be mounted to the shoulder part S, and the second assistance unit 20 may be mounted to the lower back part W. In this case, assistance can be provided in an activity of moving the shoulder part S and the lower back part W relative to each other, and thus a human can be assisted in an activity of recovering from a forward bending posture to an upright posture.

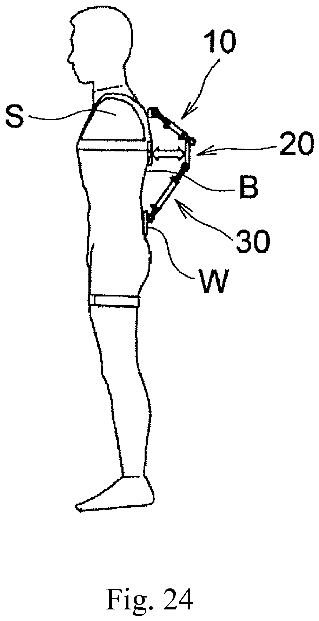

In a modification example shown in Fig. 24, a muscular strength assisting apparatus 1 may have a first assistance unit 10, a second assistance unit 20, and a third assistance unit 30. The first assistance unit 10 may be mounted to the shoulder part S, the second assistance unit 20 may be mounted to the back part B, and the third assistance unit 30 may be mounted to the lower back part W. In this case, assistance can be provided in an activity of moving the shoulder part S and the back part B relative to each other and in an activity of moving the back part B and the lower back part W relative to each other, and thus a human can be assisted in an activity of recovering from a forward bending posture to an upright posture.

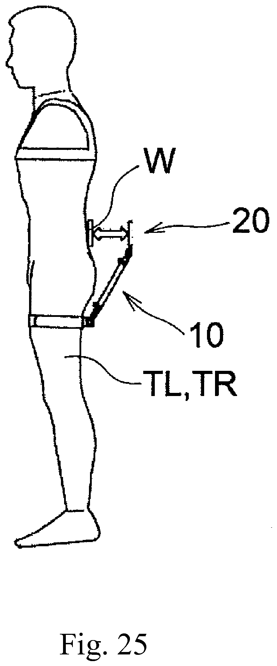

In a modification example shown in Fig. 25, a muscular strength assisting apparatus 1 may have a first assistance unit 10 and a second assistance unit 20, and a third assistance unit 30 may not be provided therein. The first assistance unit 10 may be mounted to the thigh part TL and TR, and the second assistance unit 20 may be mounted to the lower back part W. In this case, assistance can be provided in an activity of moving the lower back part W and the thigh part TL and TR relative to each other, and thus a human can be assisted in an activity of recovering from a bending posture in which the human bends his/her legs to a stretching posture.

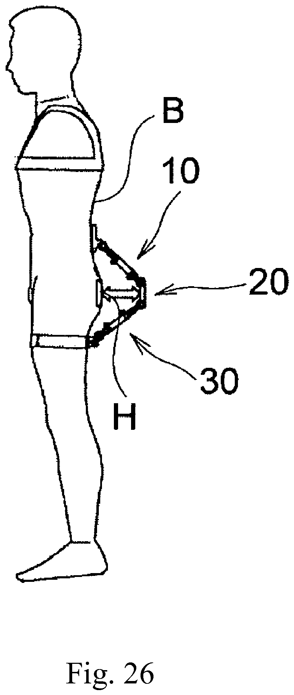

In a modification example shown in Fig. 26, a muscular strength assisting apparatus 1 may have a first assistance unit 10, a second assistance unit 20, and a third assistance unit 30. The first assistance unit 10 may be mounted to the back part B, the second assistance unit 20 may be mounted to a hip part H, and the third assistance unit 30 may be mounted to the thigh part TL and TR. In this case, assistance can be provided in an activity of moving the back part B and the hip part H relative to each other and in an activity of moving the hip part H and the thigh part TL and TR relative to each other, and thus a human can be assisted in an activity of recovering from a forward bending posture to an upright posture and in an activity of recovering from a bending posture in which the human bends his/her legs to a stretching posture.

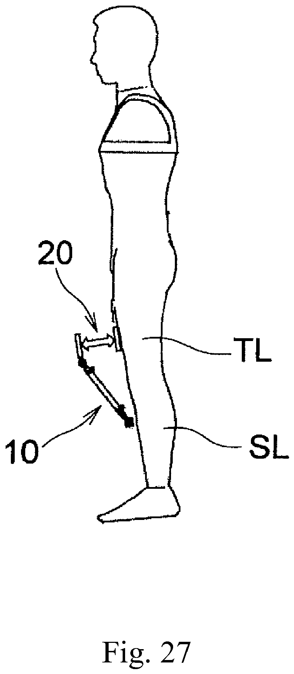

In a modification example shown in Fig. 27, a muscular strength assisting apparatus 1 may have a first assistance unit 10 and a second assistance unit 20. The first assistance unit 10 may be mounted to a lower leg part SL, and the second assistance unit 20 may be mounted to the thigh TL. In this case, assistance can be provided in an activity of moving the lower leg part SL and the thigh TL relative to each other, and thus a human can be assisted in an activity of recovering from a state where the human bends the tower leg part SL with respect to the thigh TL to a stretching posture.

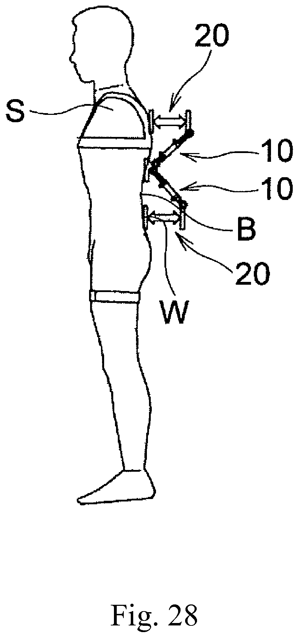

In a modification example shown in Fig. 28, two muscular strength assisting apparatuses 1 each having a first assistance unit 10 and a second assistance unit 20 may be combined together. In more detail, in one of the two muscular strength assisting apparatuses 1, the first assistance unit 10 may be mounted to the back part B, and the second assistance unit 20 may be mounted to the shoulder part S. Furthermore, in the other of the two muscular strength assisting apparatuses 1, the first assistance unit 10 may be mounted to the back part B, and the second assistance unit 20 may be mounted to the lower back part W. Further, the first assistance unit 10 of the one of the two muscular strength assisting apparatuses 1 and the first assistance unit 10 of the other muscular strength assisting apparatus 1 may be joined to each other at a mounting point with respect to the back part B. The mounting point with respect to the back part B, at which the first assistance unit 10 of the one of the two muscular strength assisting apparatuses 1 and the first assistance unit 10 of the other muscular strength assisting apparatus 1 are joined to each other, may be restricted in terms of movement in the back part B. In this case, assistance can be provided in an activity of moving the shoulder part S and the back part B relative to each other and in an activity of moving the back part B and the lower back part W relative to each other. Thus, for example, assistance can be provided in an activity of stretching the back part B in a bent state.

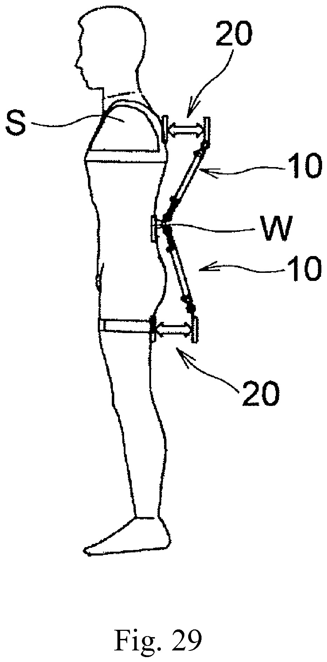

Also in a modification example shown in Fig. 29, similarly to Fig. 28, two muscular strength assisting apparatuses 1 each having a first assistance unit 10 and a second assistance unit 20 may be combined together. In this example, in one of the two muscular strength assisting apparatuses 1, the first assistance unit 10 may be mounted to the lower back part W, and the second assistance unit 20 may be mounted to the shoulder part S. Furthermore, in the other of the two muscular strength assisting apparatuses 1, the first assistance unit 10 may be mounted to the lower back part W, and the second assistance unit 20 may be mounted to the thigh part TL and TR. Further, the first assistance unit 10 of the one of the two muscular strength assisting apparatuses 1 and the first assistance unit 10 of the other muscular strength assisting apparatus 1 may be joined to each other at a mounting point with respect to the lower back part W.

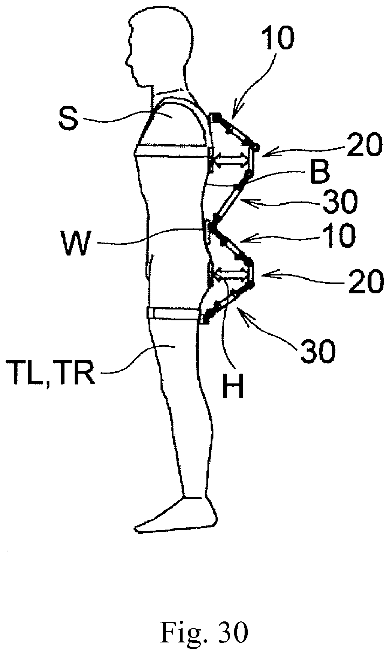

In a modification example shown in Fig. 30, two muscular strength assisting apparatuses 1 each having a first assistance unit 10, a second assistance unit 20, and a third assistance unit 30 may be combined together. In more detail, one of the two muscular strength assisting apparatuses 1 may be disposed on an upper body side, and in the upper body-side muscular strength assisting apparatus 1, the first assistance unit 10 may be mounted to the shoulder part S, the second assistance unit 20 may be mounted to the back part B, and the third assistance unit 30 may be mounted to the lower back part W. Furthermore, the other of the two muscular strength assisting apparatuses 1 may be disposed on a lower body side, and in the lower body-side muscular strength assisting apparatus 1, the first assistance unit 10 may be mounted to the lower back part W, the second assistance unit 20 may be mounted to the hip part H, and the third assistance unit 30 may be mounted to the thigh part TL and TR. Further, the third assistance unit 30 of the upper body-side muscular strength assisting apparatus 1 and the first assistance unit 10 of the lower body-side muscular strength assisting apparatus 1 may be joined to each other at a mounting point with respect to the lower back part W.

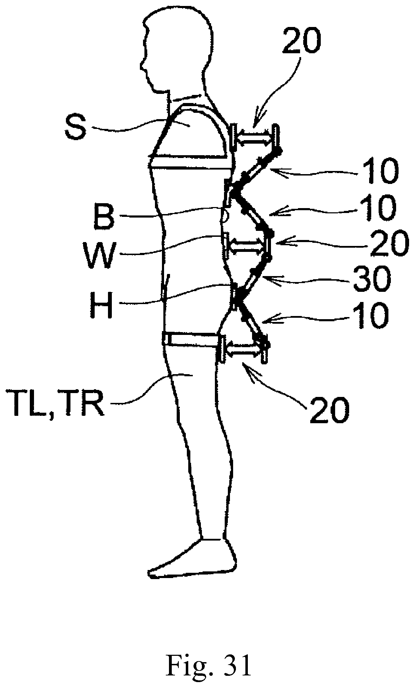

In a modification example shown in Fig. 31, a muscular strength assisting apparatus 1 having a first assistance unit 10 and a second assistance unit 20, another muscular strength assisting apparatus 1 having the first assistance unit 10, the second assistance unit 20, and a third assistance unit 30, and still another muscular strength assisting apparatus 1 having the first assistance unit 10 and the second assistance unit 20 may be combined together. In more detail, the three muscular strength assisting apparatuses 1 may be disposed in an upper body-side upper part, in an upper body-side lower part, and on a lower body side, respectively. In the muscular strength assisting apparatus 1 disposed in the upper body-side upper part, the first assistance unit 10 may be mounted to the back part B, and the second assistance unit 20 may be mounted to the shoulder part S. In the muscular strength assisting apparatus 1 disposed in the upper body-side lower part, the first assistance unit 10 may be mounted to the back part B, the second assistance unit 20 may be mounted to the lower back part W, and the third assistance unit 30 may be mounted to the hip part H. Furthermore, in the muscular strength assisting apparatus 1 disposed on the lower body side, the first assistance unit 10 may be mounted to the hip part H, and the second assistance unit 20 may be mounted to the thigh part TL and TR. Further, the first assistance unit 10 of the muscular strength assisting apparatus 1 disposed in the upper body-side upper part and the first assistance unit 10 of the muscular strength assisting apparatus 1 disposed in the upper body-side lower part may be joined to each other at a mounting point with respect to the back part B. Furthermore, the third assistance unit 30 of the muscular strength assisting apparatus 1 disposed in the upper body-side lower part and the first assistance unit 10 of the muscular strength assisting apparatus 1 disposed on the lower body side may be joined to each other at a mounting point with respect to the hip part H.

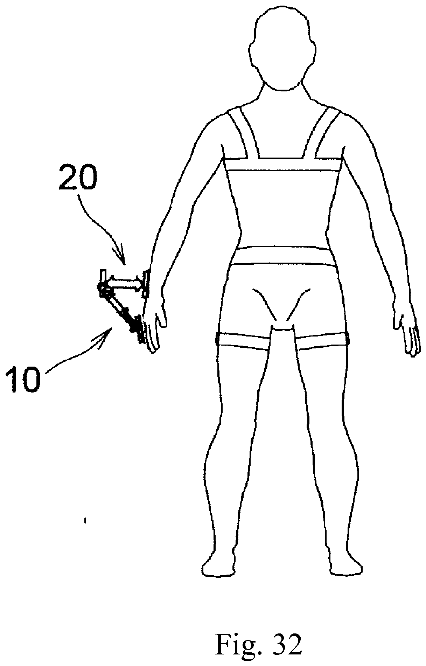

In a modification example shown in Fig. 32, a muscular strength assisting apparatus 1 may have a first assistance unit 10 and a second assistance unit 20. The first assistance unit 10 may be mounted to a palm, and the second assistance unit 20 may be mounted to a forearm part. In this case, assistance can be provided in an activity of moving the palm and the forearm part relative to each other, and thus a human can be assisted in an activity of recovering from a state where the human bends the palm with respect to the forearm part via his/her wrist.

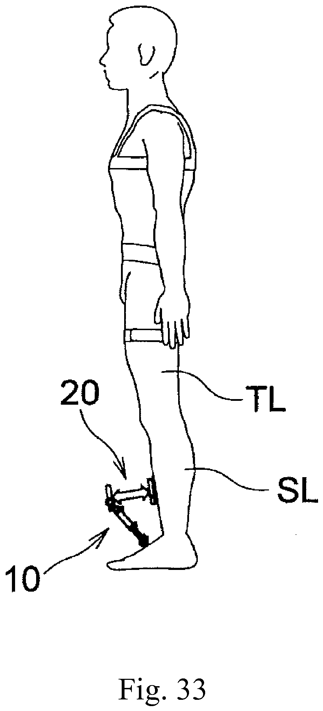

In a modification example shown in Fig. 33, a muscular strength assisting apparatus 1 may have a first assistance unit 10 and a second assistance unit 20. The first assistance unit 10 may be mounted to a foot, and the second assistance unit 20 may be mounted to a lower leg part. In this case, assistance can be provided in an activity of moving the foot and the lower leg part relative to each other, and thus a human can be assisted in an activity of recovering from a state where the human bends the foot with respect to the lower leg part via his/her ankle.

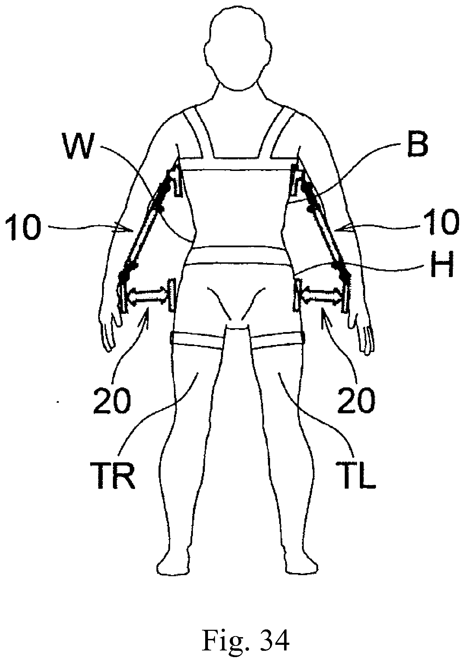

In a modification example shown in Fig. 34, muscular strength assisting apparatuses 1 each having a first assistance unit 10 and a second assistance unit 20 may be disposed on left and right sides with respect to the lower back part W. In a left-side one of the muscular strength assisting apparatuses 1, the first assistance unit 10 may be mounted to an upper left side surface of the back part B, and the second assistance unit 20 may be mounted to an upper left side surface of the left thigh TL. In a right-side one of the muscular strength assisting apparatuses 1, the first assistance unit 10 may be mounted to an upper right side surface of the back part B, and the second assistance unit 20 may be mounted to an upper right side surface of the right thigh TR. In this case, assistance can be provided in an activity of moving the lower back part W and the back part B relative to each other in a left-right direction. Thus, assistance can be provided in an activity of recovering from a curved state of the back part B to the left-right direction (a rolling direction) with respect to the lower back part W.

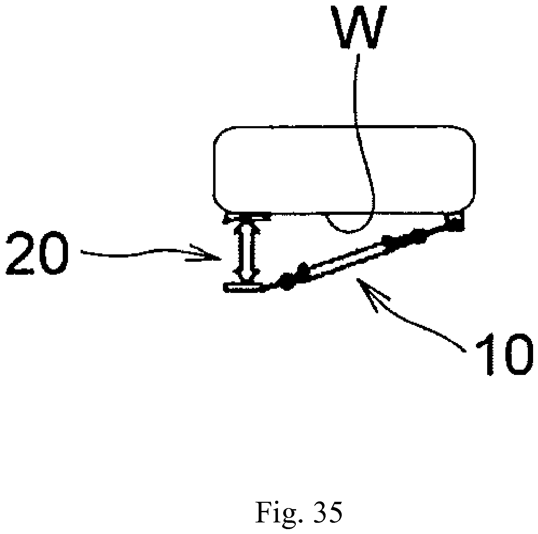

In a modification example shown in Fig. 35, a muscular strength assisting apparatus 1 having a first assistance unit 10 and a second assistance unit 20 may be disposed on the lower back part W. Fig. 35 schematically shows a transverse section of the lower back part W. The first assistance unit 10 may be mounted to a right side surface of the lower back part W. The second assistance unit 20 may be mounted to a left side surface of the lower back part W and configured to be expanded/contracted rearward. In this case, assistance can be provided in an activity of recovering from a state of rotating to a rotation direction (a yawing direction) about, as an axis, an up-down direction with respect to the lower back part W.

As thus discussed, the muscular strength assisting apparatus according to the present invention may be applicable to various body parts that move relative to each other. Other than the configurations shown in Fig. 23 to Fig. 35, the muscular strength assisting apparatus according to the present invention is applicable also to configurations in which the apparatus is mounted between a shoulder part and an upper arm part, between an upper arm part and a forearm part, and so on. That is, it may also be possible that the fist assistance unit 10 is mounted to an upper arm part, and the second assistance unit 20 is mounted to a shoulder part. Furthermore, it may also be possible that the first assistance unit 10 is mounted to a forearm part, and the second assistance unit 20 is mounted to an upper arm part. Furthermore, while in the foregoing embodiment, the third assistance unit 30 is mounted to the thigh part TL and TR of a human, it may also be possible that the third assistance unit 30 extends from a rear position of the lower back part W in the second assistance unit 20 and is mounted to a lower leg part or a foot part. In this case, when a human has taken a leg bending posture in which the human bends his/her leg part, the third actuator 31 may be operated so that a distance between the third body-side disposition portion 31A and the third outer-side disposition portion 31B is increased, and thus assistance can be provided in an activity of recovering from the bending posture to a stretching posture.

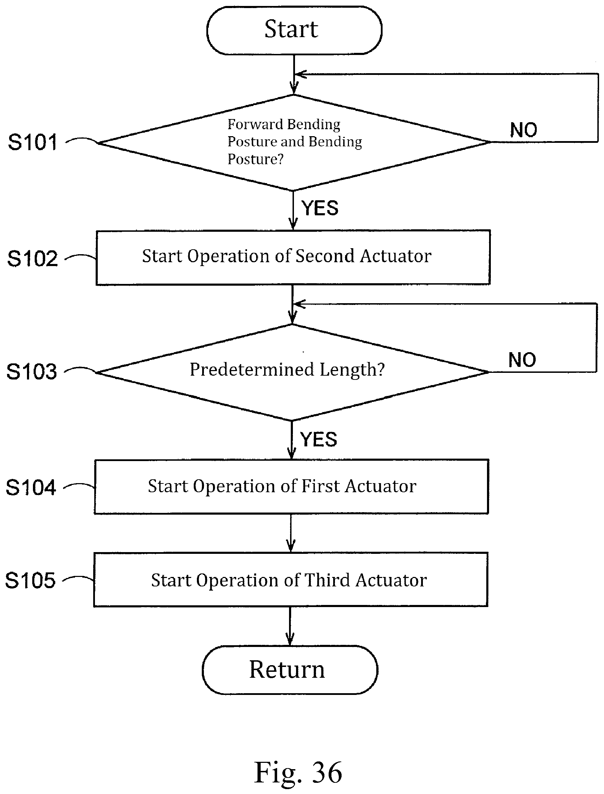

(Modification Examples of Operation) In the foregoing embodiment, in step S103 shown in Fig. 6, the controller 50 may control the status monitoring portion 52 to monitor whether or not a predetermined time has elapsed from a start of an operation of the second actuator 21, and in a case where the predetermined time has elapsed, the first actuator 11 may be operated in step S104. Instead, as shown in a flow chart in Fig. 36, it may also be possible that the status monitoring portion 52 monitors whether or not a distance between the second body-side disposition portion 21A and the second outer-side disposition portion 21B of the second actuator 21 has become a predetermined length. In this case, it may also be possible that the status monitoring portion 52 is electrically connected to a position sensor provided in the second actuator 21 and detects a length (a distance).

In a case where, as described above, it is monitored whether or not a distance between the second body-side disposition portion 21A and the second outer-side disposition portion 21B of the second actuator 21 has become a predetermined length, and timing for operating the first actuator 11 is determined based thereon, a desired moment arm can be reliably ensured

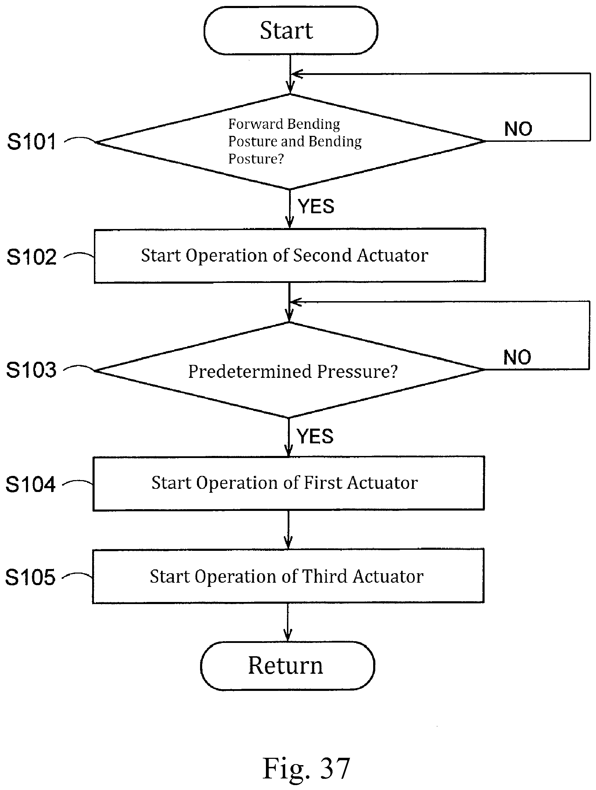

Furthermore, as shown in a flow chart in Fig. 37, it may also be possible that the status monitoring portion 52 monitors whether or not a pressure inside the bellows portion 21C joining the second body-side disposition portion 21A to the second outer-side disposition portion 21B of the second actuator 21 has become a predetermined pressure. In this case, it may also be possible that the status monitoring portion 52 is electrically connected to a pressure sensor provided in the second actuator 21 and detects a pressure.

In a case where, as described above, it is monitored whether or not a pressure inside the bellows portion 21C has become a predetermined pressure, and timing for operating the first actuator 11 is determined based thereon, a pressure inside the bellows portion 21C may be prevented from becoming excessive.

It may also be possible that, in step S103, the time, the length, and the pressure are all monitored, and when all conditions are satisfied, the first actuator 11 is operated in step S104.

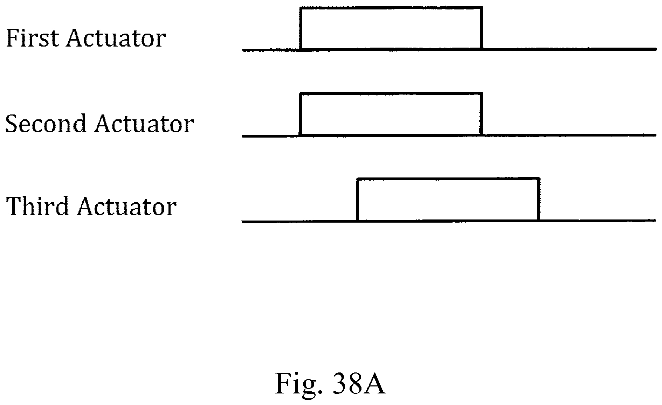

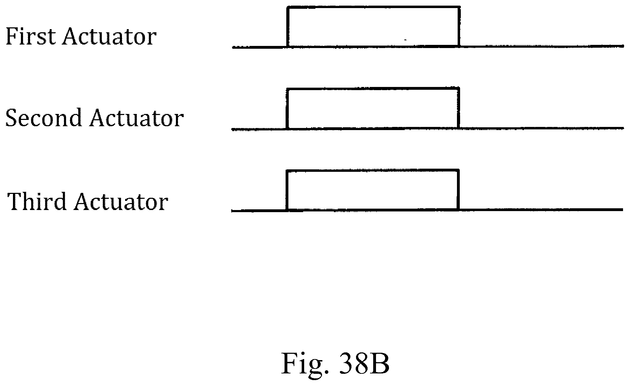

Furthermore, in the foregoing embodiment, when a human has taken a forward bending posture and a leg bending posture, the second actuator 21 may be operated, and then, the first actuator 11 may be operated, after which the third actuator 31 may be operated It may also be possible that this timing is changed, for example, as shown in Fig. 38A. That is, in an example shown in Fig. 38A, the first actuator 11 and the second actuator 21 may be operated at the same timing, and then the third actuator 31 may be operated Furthermore, for reference, it may also be possible that, as shown in Fig. 38B, the first actuator 11, the second actuator 21, and the third actuator 31 are all operated at the same timing.

Furthermore, the foregoing embodiment has explained that examples of the body posture detection sensor 14 that detects a forward bending posture of a human and the leg posture detection sensor 34 that detects a leg bending posture of a human may include an electromyography sensor, an angle sensor, an acceleration sensor, a strain gauge, and the like. However, not only these types of sensors, a pressure sensor provided in a glove, a pressure sensor that detects a pressure applied on a foot, or the like can also be used as a sensor that detects a human posture, In a case of using a pressure sensor, based on a pressure value detected, it can be detected whether or not each of the assistance units 10 to 30 has a force sufficient for providing assistance, and thus based on the pressure value, for examples, an additional amount of air may be supplied to each of the assistance units 10 to 30 so that effective assistance can be implemented Furthermore, an electromyography sensor, an acceleration sensor, or the like can also be used to detect whether or not each of the assistance units 10 to 30 has a force sufficient for providing assistance.

### LIST OF REFERENCE NUMBERS

- 1: muscular strength assisting apparatus
- 10: first assistance unit
- 11: first actuator
- 11A: first body-side disposition portion
- 11B: first outer-side disposition portion
- 14: body posture detection sensor
- 20: second assistance unit
- 21: second actuator
- 21A: second body-side disposition portion
- 21B: second outer-side disposition portion
- 30: third assistance unit
- 31: third actuator
- 31A: third body-side disposition portion
- 31B: third outer-side disposition portion
- 34: leg posture detection sensor
- 40: control unit
- 50: controller

## Claims

1. A muscular strength assisting apparatus, comprising:
a first assistance unit having a first actuator including a first body-side disposition portion mounted to a first part of a body and a first outer-side disposition portion separable from and contactable with the first body-side disposition portion, the first actuator being capable of changing a distance between the first body-side disposition portion and the first outer-side disposition portion; and
a second assistance unit having a second actuator including a second body-side disposition portion mounted to a second part of the body and a second outer-side disposition portion separable from and contactable with the second body-side disposition portion, the second actuator being capable of changing a distance between the second body-side disposition portion and the second outer-side disposition portion, the second outer-side disposition portion being mountable to the first outer-side disposition portion,
wherein the first assistance unit is mounted to the first part and the second assistance unit is mounted to the second part so that a direction connecting the second body-side disposition portion to the second outer-side disposition portion of the second actuator is non-parallel to a direction connecting the first body-side disposition portion to the first outer-side disposition portion.

2. The muscular strength assisting apparatus according to claim 1, wherein, in a state where the second actuator is stopped from operating, the second assistance unit is capable of maintaining a distance between the second body-side disposition portion and the second outer-side disposition portion at a predetermined length.

3. The muscular strength assisting apparatus according to claim 1 or 2, wherein, in a state where the first actuator is stopped from operating, the first assistance unit is capable of maintaining a distance between the first body-side disposition portion and the first outer-side disposition portion at a predetermined length.

4. The muscular strength assisting apparatus according to any one of claims 1 to 3, wherein the first part represents a shoulder part or a thigh part, and the second part represents a lower back part.

5. The muscular strength assisting apparatus according to any one of claims 1 to 4, further comprising:
a control device for controlling the second actuator to operate so that, before the first actuator starts to operate, the second outer-side disposition portion of the second actuator moves away from the second part.

6. The muscular strength assisting apparatus according to any one of claims 1 to 3, further comprising:
a third assistance unit having a third actuator including a third body-side disposition portion mounted to a third part of the body and a third outer-side disposition portion separable from and contactable with the third body-side disposition portion, the third actuator being capable of changing a distance between the third body-side disposition portion and the third outer-side disposition portion, the third outer-side disposition portion being mountable to the second outer-side disposition portion,
wherein the third assistance unit is mounted to the third part so that a direction connecting the second body-side disposition portion to the second outer-side disposition portion of the second actuator is non-parallel to a direction connecting the third body-side disposition portion to the third outer-side disposition portion of the third actuator.

7. The muscular strength assisting apparatus according to claim 6, wherein, in a state where the third actuator is stopped from operating, the third assistance unit is capable of maintaining a distance between the third body-side disposition portion and the third outer-side disposition portion at a predetermined length.

8. The muscular strength assisting apparatus according to claim 6 or 7, wherein the first part represents a shoulder part, the second part represents a lower back part, and the third part represents a thigh part.

9. The muscular strength assisting apparatus according to claims 6 to 8, further comprising:
a control device for controlling the second actuator to operate so that, before the first actuator and the third actuator start to operate, the second outer-side disposition portion of the second actuator moves away from the second part.
